# EUROPEAN PATENT SPECIFICATION

(11) **EP 2 914 606 B1**
(45) Date of publication and mention of the grant of the patent: **03.07.2019**
(21) Application number: 13850907.0
(22) Date of filing: 05.11.2013
(51) Int. Cl.: C07F 9/50, C07B 61/00, C07F 15/00

(54) **COMPLEXES OF PHOSPHINE LIGANDS COMPRISING A CARBA-CLOSO-DODECABORATE SUBSTITUENT**
KOMPLEXE AUS PHOSPHINLIGANDEN MIT EINEM CARBA-CLOSO-DODECABORATSUBSTITUENT
COMPLEXES DE LIGANDS DE PHOSPHINE COMPRENANT UN SUBSTITUANT CARBA-CLOSO-DODÉCABORATE

(30) Priority: 05.11.2012 US 201261722740 P
(43) Date of publication of application: 09.09.2015
(73) Proprietor: The Regents of the University of California, Oakland, CA 94607 (US)
(72) Inventor: LAVALLO, Vincent, Oakland, CA 94607 (US)
(74) Representative: J A Kemp
(86) International application number: PCT/US2013/068581
(87) International publication number: WO 2014/071401

(56) References cited:
- ALEXANDER HIMMELSPACH ET AL: "Microwave-Assisted Kumada-Type Cross-Coupling Reactions of Iodinated Carba- closo -dodecaborate Anions", INORGANIC CHEMISTRY, vol. 51, no. 4, 20 February 2012 (2012-02-20), pages 2679-2688, XP055254476, EASTON, US ISSN: 0020-1669, DOI: 10.1021/ic202638k
- DATABASE CA [Online] CHEMICAL ABSTRACTS SERVICE, COLUMBUS, OHIO, US; 1993, JELINEK, TOMAS ET AL: "New weakly coordinating anions. 2. Derivatization of the carborane anion CB11H12-", XP002756108, retrieved from STN Database accession no. 1993:408855 & JELINEK T ET AL: "NEW WEAKLY COORDINATING ANIONS. 2. DERIVATIZATION OF THE CARBORANE ANION CB11H12-", INORGANIC CHEMISTRY, AMERICAN CHEMICAL SOCIETY, EASTON, US, vol. 32, no. 10, 1 May 1993 (1993-05-01), pages 1982-1990, XP002914226, ISSN: 0020-1669, DOI: 10.1021/IC00062A018
- DATABASE CA [Online] CHEMICAL ABSTRACTS SERVICE, COLUMBUS, OHIO, US; 22 May 2013 (2013-05-22), DRISCH, MICHAEL ET AL: "Carba-closo-dodecaborate Anions with Cluster Carbon-Phosphorous Bonds", XP002756109, retrieved from STN Database accession no. 2013:791357 & Michael Drisch ET AL: "Carba- closo -dodecaborate Anions with Cluster Carbon-Phosphorous Bonds", ZEITSCHRIFT FUR ANORGANISCHE UND ALLGEMEINE CHEMIE., vol. 639, no. 7, 22 May 2013 (2013-05-22), pages 1134-1139, XP055261670, DE ISSN: 0044-2313, DOI: 10.1002/zaac.201300130
- VINCENT LAVALLO ET AL: "Perhalogenated Carba- closo -dodecaborate Anions as Ligand Substituents: Applications in Gold Catalysis", ANGEWANDTE CHEMIE INTERNATIONAL EDITION, vol. 52, no. 11, 13 February 2013 (2013-02-13), pages 3172-3176, XP055261667, DE ISSN: 1433-7851, DOI: 10.1002/anie.201209107
- ROSARIO NUUEZ ET AL.: 'Coordination of the nido-carboranyldiphosphine liga nd to ruthenium(II): the first example of the tricoordinating capacity of th e 7,8-(PPh2)2-7,8-C2B9H10 moiety' APPL. ORGANOMETAL. CHEM. vol. 17, 2003, pages 509 - 717, XP055254128
- MATTHEW J. NAVA ET AL.: 'High Yield C-Derivatization of Weakly Coordinating Carborane Anions' INORG CHEM. vol. 49, no. 11, 07 June 2010, pages 4726 - 4728, XP055251777
- WEIXING GU ET AL.: 'Improved methods for the halogenation of the [HCB11H11] - anion' CHEM. COMMUN. vol. 46, 2010, pages 2820 - 2822, XP055254489
- ALEXANDER HIMMELSPACH ET AL.: 'Microwave-Assisted Kumada-Type Cross-Couplin g Reactions of Iodinated Carba-closo-dodecaborate Anions' INORG. CHEM. vol. 51, no. 4, 26 January 2012, pages 2679 - 2688, XP055254476
- A A Tyutyunov ET AL: "Phosphorus derivatives of carboranes as ligands for Pddcatalyzed crossscoupling reactions", Russian Chemical Bulletin International Edition, 1 November 2008 (2008-11-01), pages 2307-2310, XP055477057, Retrieved from the Internet: URL:https://link.springer.com/content/pdf/ 10.1007/s11172-008-0326-y.pdf

## Description

### FIELD OF THE INVENTION

This invention relates to transition metal catalysts and their use in catalytic reactions such as, but not limited to, olefin polymerization.

### BACKGROUND OF THE INVENTION

Modern transition metal catalysis is based, in part, on metal complexes that include a diverse range of ligand frameworks, such as bulky R-groups which influence the catalytic activity of these complexes. These types of sterically demanding ligands kinetically protect the active metal center and promote substrate exchange and low coordination, both of which are necessary processes for high turnover numbers (TON). These ligands also influence the metal center's selectivity for product formation and substrate consumption.

Of the many ligands used in modern metal catalysis, dicarba-closo-dodecaborane (C₂B₁₀) clusters (M. Scholz, et al., Chem. Rev. 2011, 111, 7035-7062; N. Fey, et al., Organometallics 2012, 31, 2907-2913; A. M. Spokoyny, et al., Nature Chem. 2011, 3, 590-596; J. I. van der Vlugt, Angew. Chem. 2010, 122, 260-263; Angew. Chem. Int. Ed. 2010, 49, 252-255) are not commonly used. These boron containing ligands have not been employed much in the relevant field because of potential negative side reactions, such as B-H activation and cyclometalation. For these and related reasons, such as intramolecular reactions, the synthetic utility of carborane bearing ligands in the area of catalysis has been limited.

A. A. Tyutyunov et al (Russian Chemical Bulletin International Edition (2008-11-01), pp 2307-2310) describe phosphorus derivatives of carboranes as ligands for Pd-catalyzed cross-coupling reactions.

Complexes that contain ligands functionalized with related carba-closo-dodecaborate anions (CB₁₁⁻) directly bound to the coordinating atom via the carborane hypercarbon have not been reported (S. Körbe, et al., Chem. Rev. 2006, 106, 5208-5249).

Although similar in size to their neutral dicarba-cousins (C₂B₁₀), isoelectronic (CB₁₁-) clusters have significantly different properties. The negative charge of these clusters is delocalized throughout the 12 cage atoms, resulting in a very weakly coordinating anion. This weak coordinative ability can be enhanced by substitution of some or all of the B-H vertices by alkyl or halo-groups. In the case of alkyl substitution, the cluster becomes more reactive towards substitution and oxidation chemistry. On the other hand, exhaustive halogenation of the cluster's boron vertices introduces electron withdrawing substituents that enhance the anion's inherent weak coordinative ability and also confers these molecules with inertness to certain chemicals and reactions. For example, some perhalogenated carborane counteranions are observed to form isolable salts with potent oxidants such as C₆₀⁺ and CH₃⁺.

There is currently a need, for example, for single component gold catalysts which offer a number of advantages compared to traditional two component systems that utilize Ag⁺ or strong Bronsted acid additives. These additives not only increase the cost of the systems, but can also induce side reactions or different reactivity all together. Accordingly, there exists a need in the field to which the instant invention relates regarding the development and use of novel bulky ligands and their associated transition metal complexes. Surprisingly, the instant invention meets these as well as other needs in the relevant field.

### BRIEF SUMMARY OF THE INVENTION

In one aspect, the instant application sets forth a composition comprising a transition metal and at least one ligand comprising a carba-closo-dodecaborate substituent.

The present invention provides a ligand having the following structure:

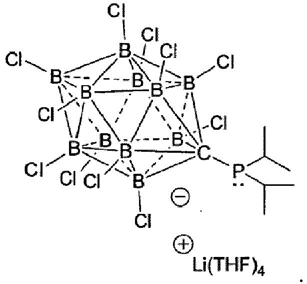

The present invention also provides a complex having a structure selected from:

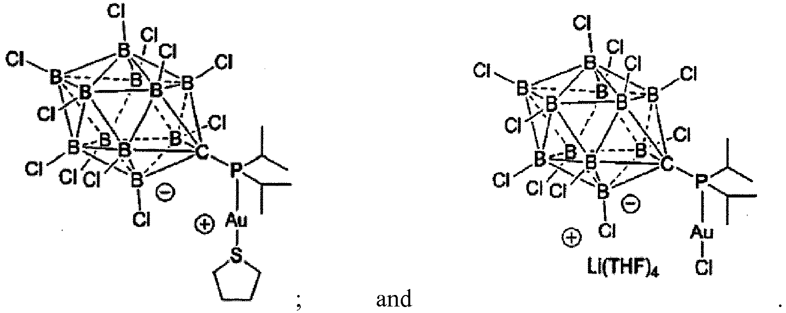

The present invention also provides the use of said complexes in a method of catalysing a chemical reaction, optionally wherein the chemical reaction is hydroamination.

The present invention also provides a method for catalysing a chemical reaction comprising contacting said complexes with suitable reagents for the chemical reaction to occur, optionally wherein the chemical reaction is a hydroamination reaction.

In a further aspect, the instant application provides a methods of catalyzing chemical reactions, wherein the chemical reactions include, but are not limited to, olefin polymerization, hydroaddition, hydroamination, cross coupling, olefin metathesis hydroformylation, hydrogenation, hydroaminomethylation.

### BRIEF DESCRIPTION OF THE DRAWINGS

Figure 1 shows a synthetic procedure for preparing a phosphine ligand containing a carba-closo-dodecaborate ligand substituent in accordance with the present disclosure.
Figure 2 shows a solid state structure of an phosphine ligand containing a carba-closo-dodecaborate ligand substituent 2, wherein the hydrogen atoms are omitted for clarity. Thermal ellipsoids drawn at the 50% probability level.
Figure 3 shows the synthesis of complexes 3(THT) and 3(LiCl), top left and right respectively. Solid state structure of 3(THT), bottom left. Solid state structure of 3(LiCl), bottom right. Selected bond lengths for 3(LiCl); P-Au = 2.2477(12), P-C1 = 1.943(5), P-C2 = 1.858(6), P-C3 = 1.864(5), Au-Cl = 2.2883(12). Bond lengths in Å, thermal ellipsoids drawn at the 50% probability level, hydrogen atoms in both 3(THT) and 3(LiCl) omitted for clarity.

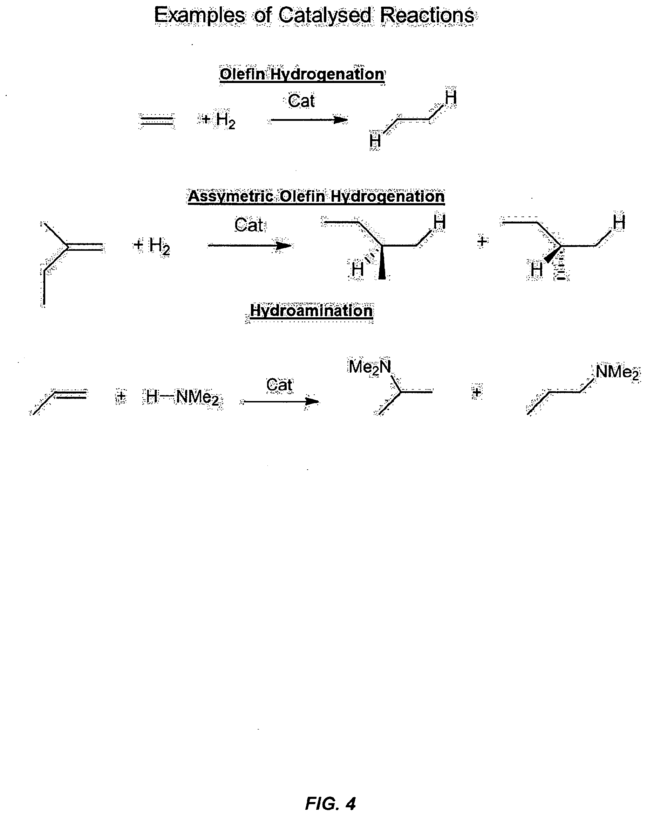

Figure 4 shows non-limiting examples of the types of reactions which the compounds of the instant disclosure are suitable for catalyzing.

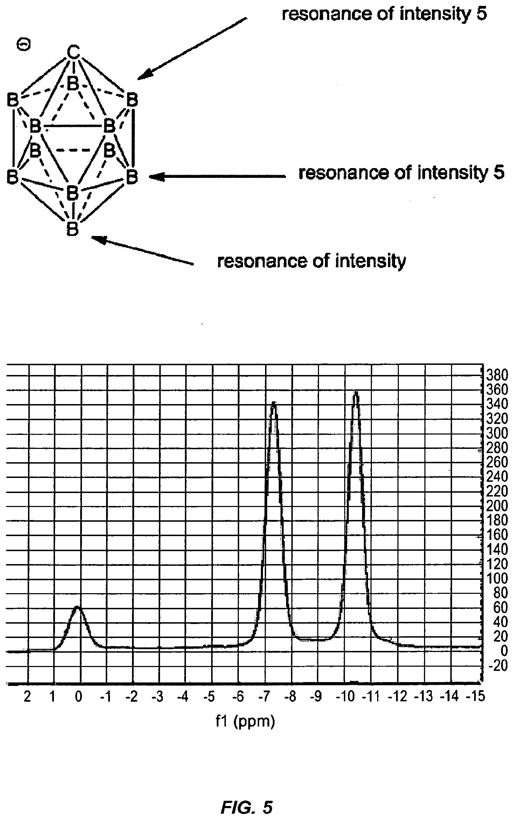

Figure 5 shows an example of Boron NMR.

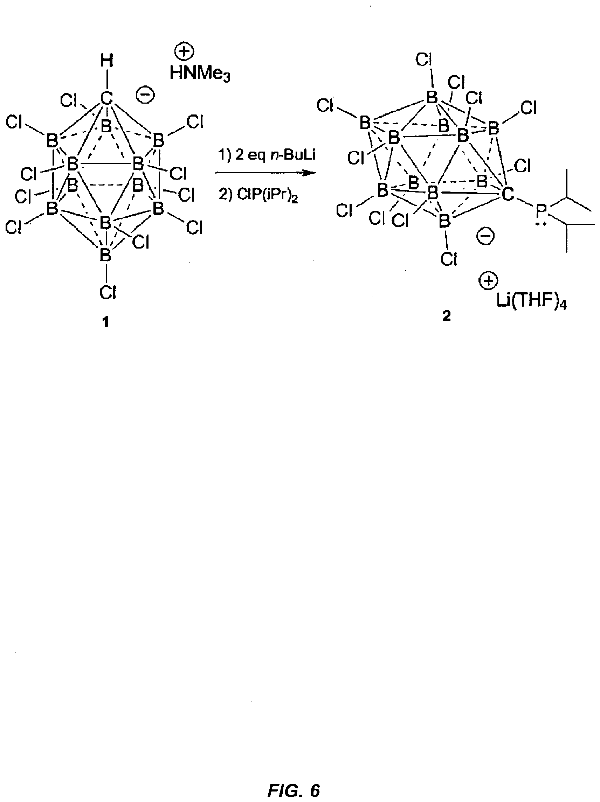

Figure 6 shows a synthetic procedure for preparing a ligand suitable for use in a complex with a transition metal.

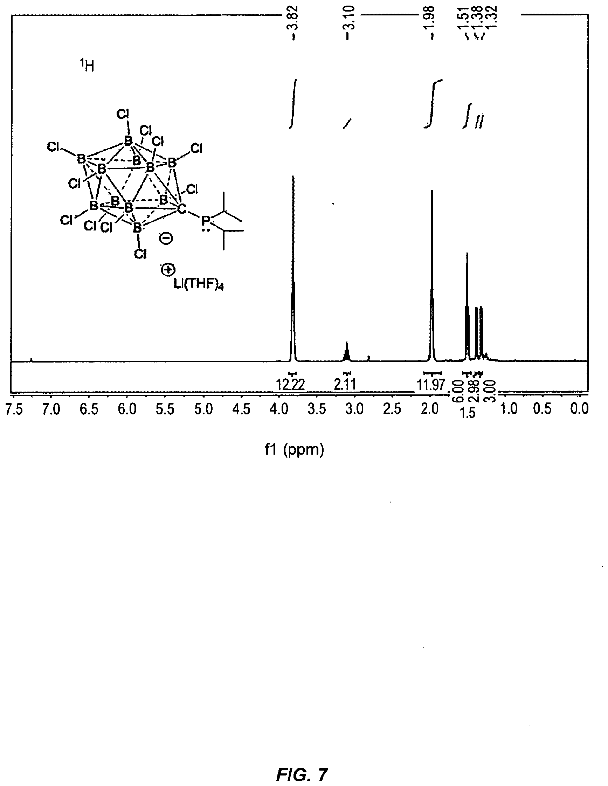

Figure 7 shows ¹H NMR data for the complex illustrated therein.

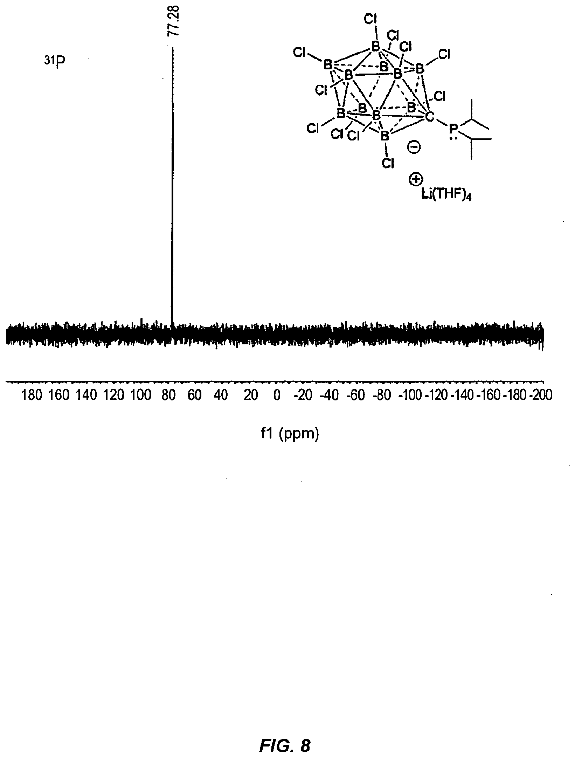

Figure 8 shows ³¹P NMR data for the complex illustrated therein.

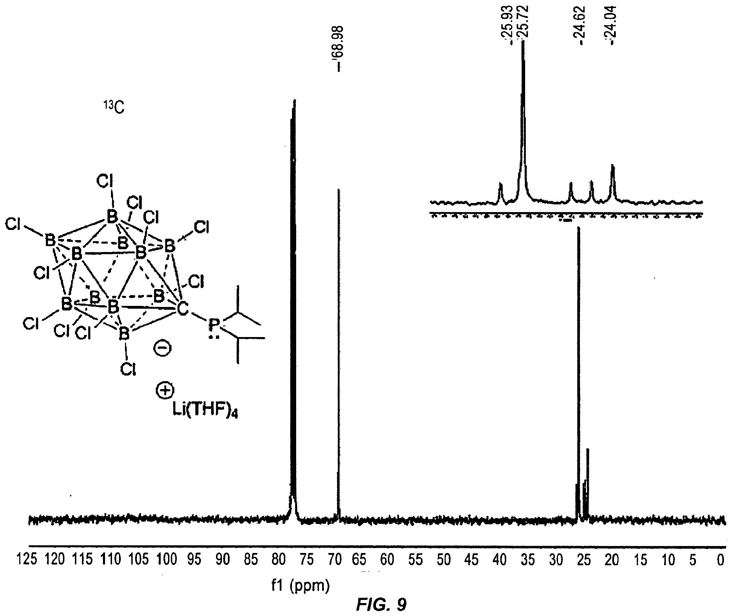

Figure 9 shows ¹³C NMR data for the complex illustrated therein.

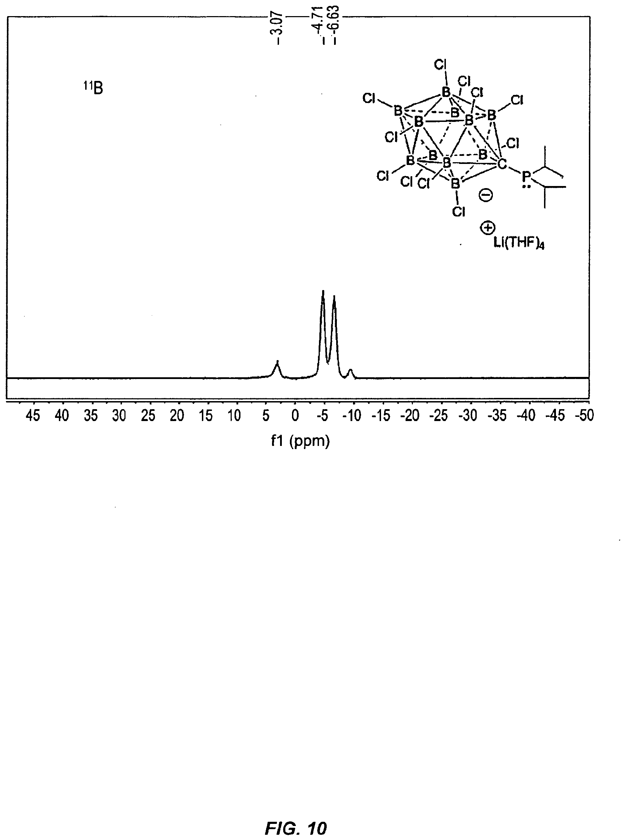

Figure 10 shows ¹¹B NMR data for the complex illustrated therein.

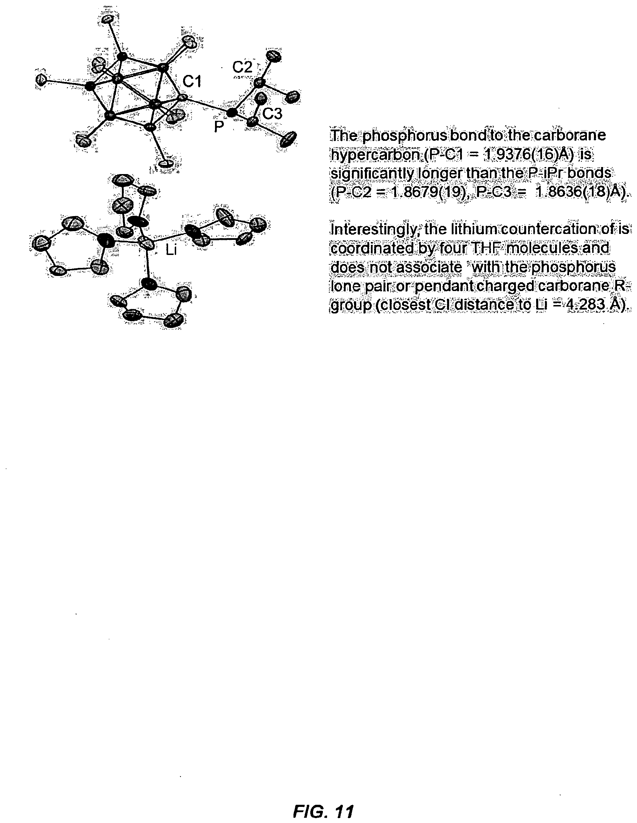

Figure 11 shows a solid state structure of a phosphine complex.

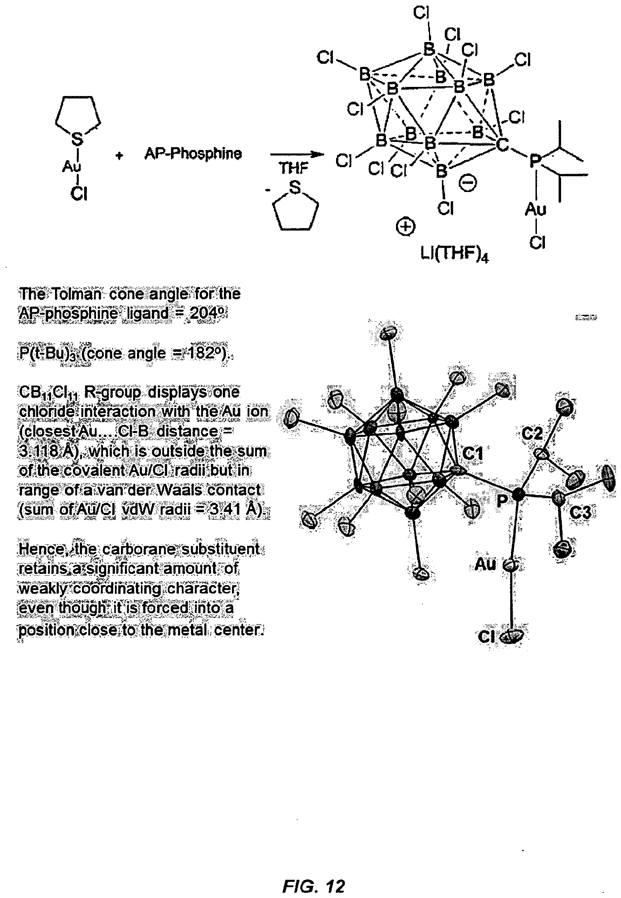

Figure 12 Figure 2 shows a solid state structure of a phosphine Au complex.

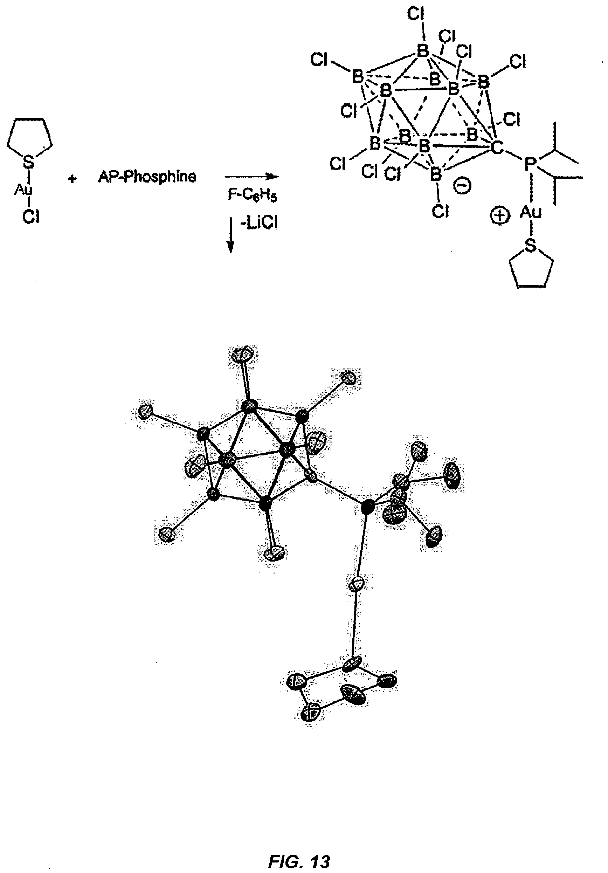

Figure 13 shows a synthetic procedure for preparing a Au complex.

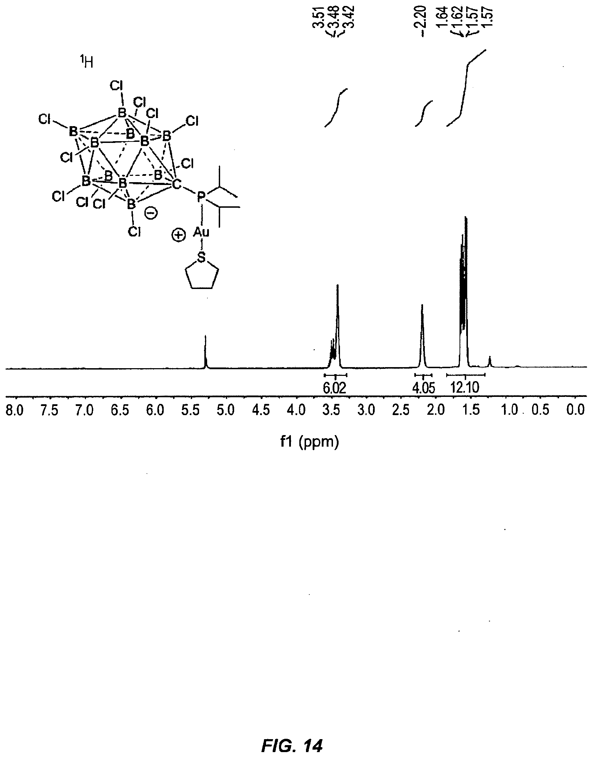

Figure 14 shows ¹H NMR data for the complex illustrated therein.

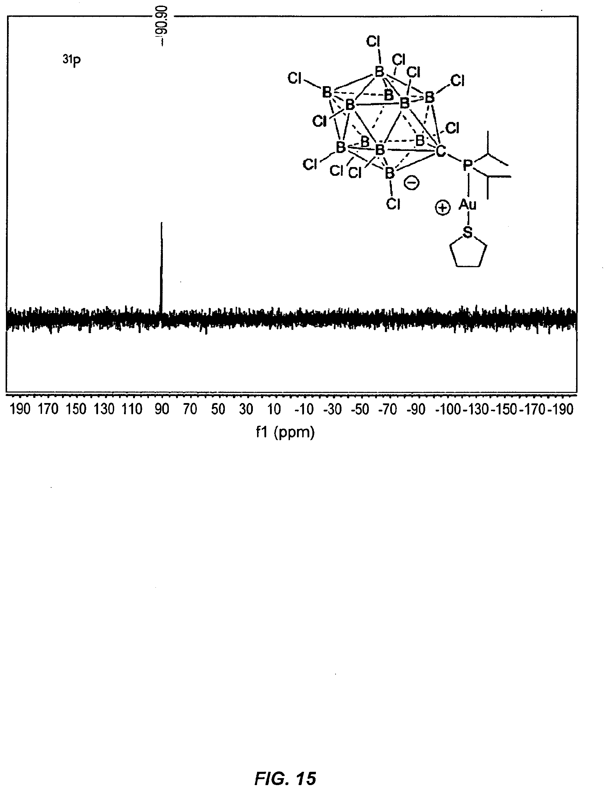

Figure 15 shows ³¹P NMR data for the complex illustrated therein.

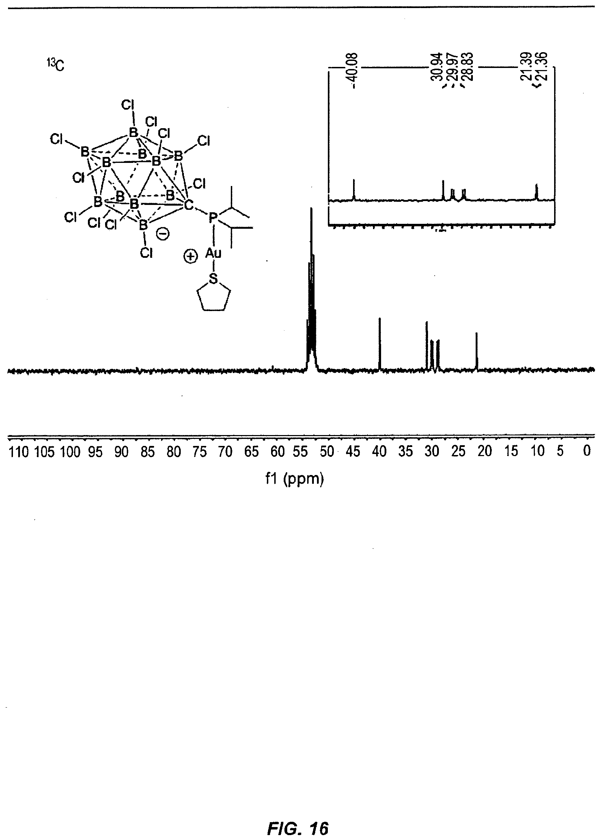

Figure 16 shows ¹³C NMR data for the complex illustrated therein.

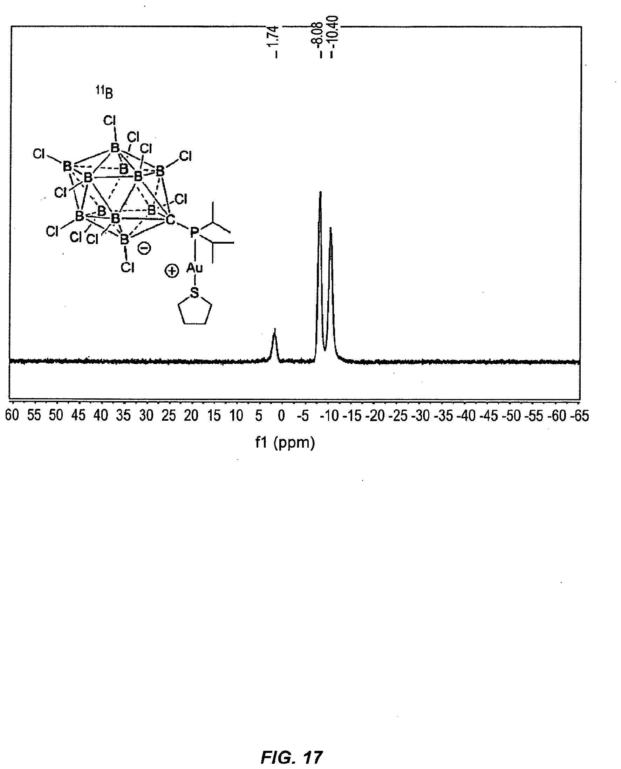

Figure 17 shows ¹¹B NMR data for the complex illustrated therein.

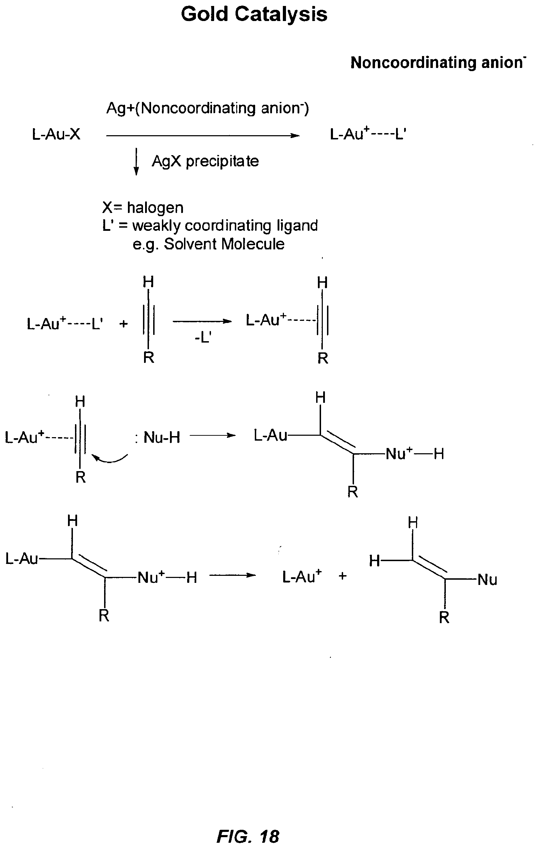

Figure 18 shows example reactions suitable for catalysis by Au complexes.

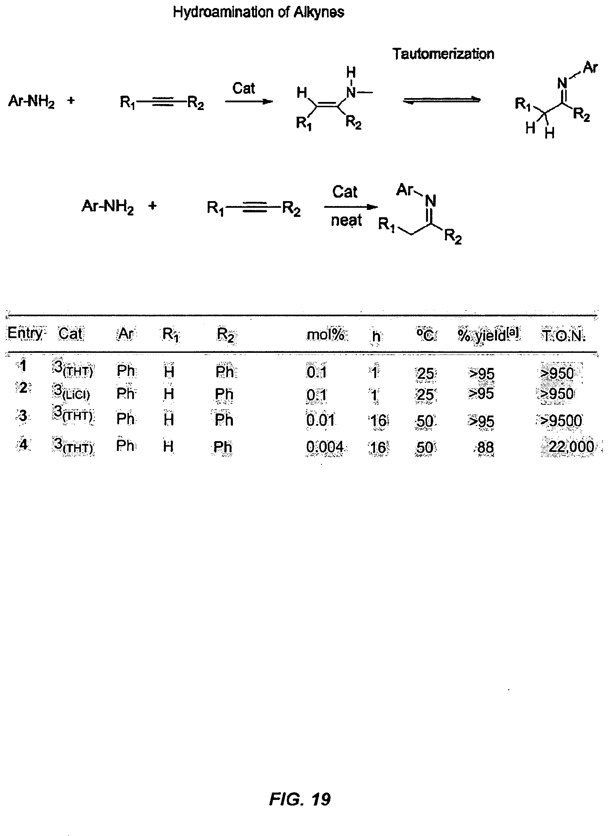

Figure 19 shows hydroamination of alkynes results observed using catalysts of the present invention.

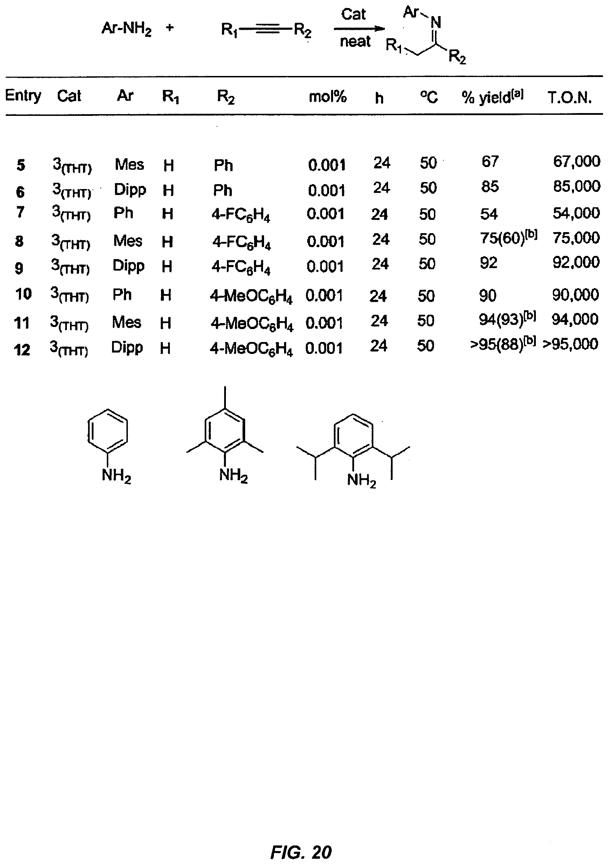

Figure 20 shows hydroamination of alkynes results observed using catalysts of the present invention.

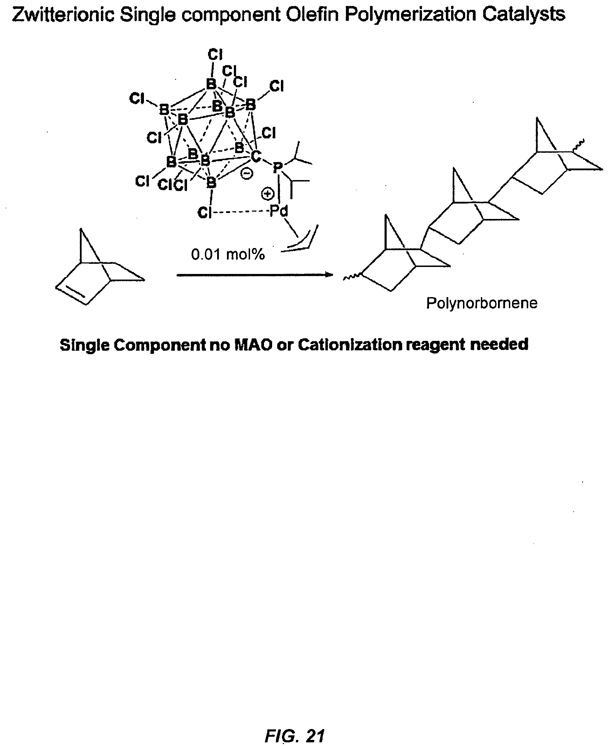

Figure 21 shows an example of olefin polymerization.

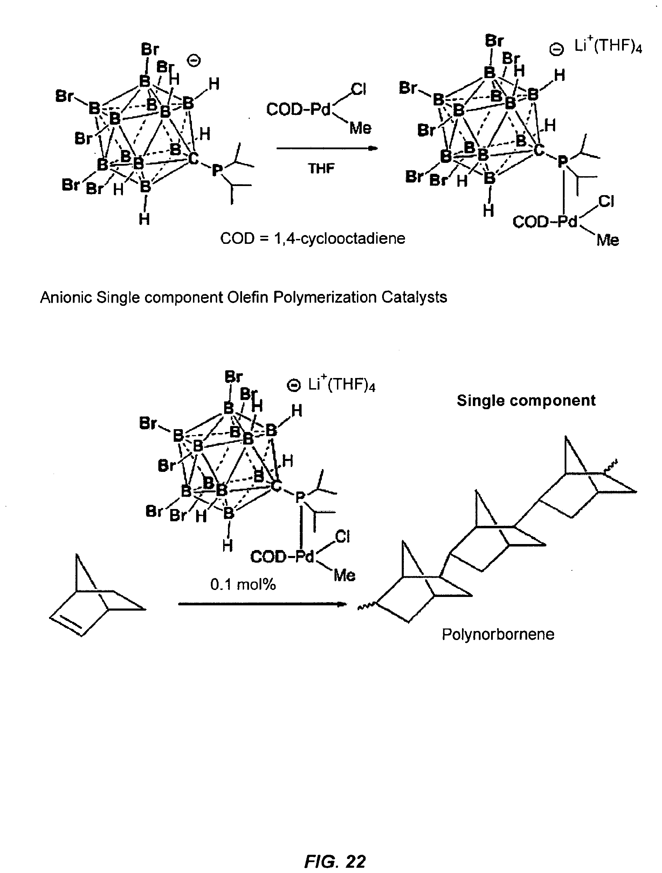

Figure 22 shows an example of olefin polymerization.

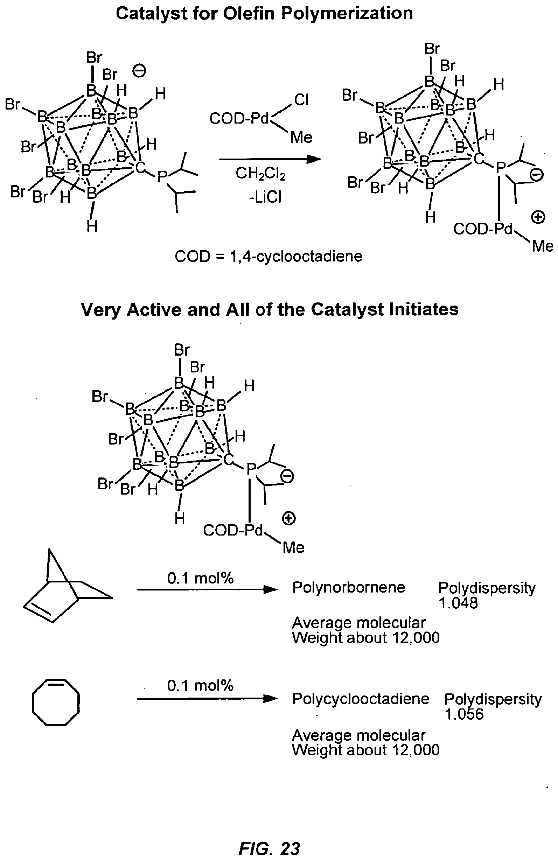

Figure 23 shows an example of olefin polymerization.

### DETAILED DESCRIPTION OF THE INVENTION GENERAL

The present disclosure provides complexes, such as zwitterionic iridium complex of a phosphine bearing carba-*closo*-dodecaborate anion ligand substituents.

The present disclosure also provides methods of making these complexes as well as methods of using these complexes as catalysts.

Whereas as other catalysts suffer from a variety of disadvantages, the instant compounds and complexes advantageously include ligands with very stable and unreactive 3-dimensionally anionic carborane clusters.

The instant compounds and complexes are less likely to be made inactive during catalysis compared to standard catalysts that feature ligands with only hydrocarbon substituents.

### DEFINITIONS

As used herein, the term "zwitterionic" or "zwitterion" refers to a neutrally charged compound having both positive and negative charged groups therein.

As used herein, the term "halide," refers to an anion of F, Cl, Br, or I. "Halogen" as used herein includes F, Cl, Br, or I.

As used herein, the term "alkyl," refers to a hydrocarbon group derived from an alkane by removing one hydrogen atom. Examples of alkyl include, but are not limited to, methyl, ethyl, isopropyl, n-propyl, n-butyl, t-butyl, isopentyl, and n-pentyl. Alkyl can include any alkyl group having from about 1 to about 16 carbon atoms.

As used herein, the term "alkoxy" refers to a group having the formula -O-R, wherein R is alkyl or aryl.

As used herein, the term "aryl" refers to a substituent derived from an aromatic compound by removing one hydrogen atom. Examples of aryl include, but are not limited to, phenyl, mesityl, 2,6,-diisopropylphenyl, napthyl, and benzyl.

As used herein, the term "leaving group," refers to group that is displaced by a nucleophile. Examples include, but are not limited to, halides and sulfonate esters, *e.g.,* tosylate.

As used herein, chelating refers to a ligand that bonds to a metal center through more than one bond. Examples include bidentate and tridentate chelating ligands such as, but not limited to, EDTA and citric acid.

As used herein, the term "phosphine ligand," refers to a ligand that includes a phosphine bond. In the relevant field to which the instant invention pertains, phosphines are derivatives of PH₃ in which one, two or three hydrogens are replaced by, for example, alkyl or aryl substituents. Examples of phosphines include, but are not limited to, triphenylphosphine.

As used herein, the term "silyl" refers to a group having the formula -Si-R₃, wherein R is alkyl or aryl.

As used herein, the term "carba-closo-dodecaborate substituent" refers to a compound having the following formula -CB₁₁R¹₁₁⁻¹ where the carbon atom is attached to a phosphine ligand as depicted below: 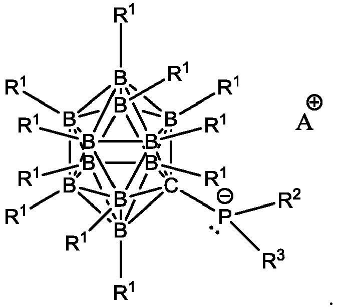 R¹ is selected from H, halogen, alkyl, aryl, silyl, or alkoxy. R² and R³ are each independently alkyl, aryl, alkoxy, or NR⁴R⁵. R⁴ and R⁵ are each independently alkyl, aryl, or alkoxy. A is a cation *[e.g.,* Li, Na, K, Cs, HN(alkyl)₃, or N(alkyl)].

### COMPLEXES

In certain aspects, the present disclosure further provides compounds and complex, e.g., zwitterionic iridium complex of a phosphine comprising the carba-*closo*-dodecaborate anion as a ligand substituent. In some aspects, when the transition metal, e.g., iridium, is bonded directly to a phosphine ligand, the CB₁₁H₁₁⁻R⁻ group engages in agostic-like bonding, utilizing the B-H bonds adjacent to the carbon atom in the cluster. Evidence for the interactions is observed in solution by variable temperature NMR and also in the solid state by a single crystal X-ray diffraction study. A significant lengthening of a *trans*-olefin C-C bond suggests this ligand substituent has a pronounced *trans*-influence, which is in contrast to the unfunctionalized weakly coordinating HCB₁₁H₁₁⁻ anion.

In some aspects, the instant application provides a composition including a transition metal and at least one ligand that includes at least 1 carba-closo-dodecaborate substituent.

In certain aspects, the instant application provides a ligand having the following structure: 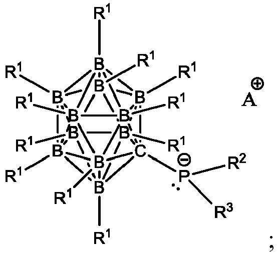 R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. A is a cation. In certain aspects, A is selected from Li, Na, K, Cs, HN(alkyl)₃, or N(alkyl)₄.

In some aspects of the above formula, R¹ is H. In other aspects R¹ is F. In some other aspects, R¹ is Cl. In other aspects, R¹ is Br. In certain aspects, R¹ is methyl. In other aspects, R¹ is ethyl. In some aspects, R¹ is propyl (e.g., n-propyl, i-propyl). In other aspects R¹ is butyl (e.g., n-butyl, i-butyl). In some other aspects, R¹ is pentyl (n-pentyl, i-pentyl , cyclopentyl). In other aspects, R¹ is hexyl (n-hexyl, cyclohexyl). In certain aspects, R¹ is octyl. In other aspects, R¹ is nonyl. In yet others, R¹ is dodecyl.

In some aspects of the above formula, R¹ is phenyl, benzyl, or napthyl. In other aspects R¹ is phenyl. In some other aspects, R¹ is benzyl. In other aspects, R¹ is napthyl. In certain aspects, R¹ is tolulyl. In other aspects, R¹ is methoxy. In some aspects, R¹ is ethoxy. In other aspects R¹ is propoxy. In some other aspects, R¹ is butoxy. In other aspects, R¹ is trimethylsilyl. In certain aspects, R¹ is triethylsilyl.

In certain aspects, R² is methyl. In other aspects, R² is ethyl. In some aspects, R² is propyl (e.g., n-propyl, i-propyl). In other aspects R² is butyl (e.g., n-butyl, i-butyl). In some other aspects, R² is pentyl (n-pentyl, i-pentyl , cyclopentyl). In other aspects, R² is hexyl (n-hexyl, cyclohexyl). In certain aspects, R² is octyl. In other aspects, R² is nonyl. In yet others, R² is dodecyl.

In some aspects of the above formula, R² is phenyl, benzyl, or napthyl. In other aspects R² is phenyl. In some other aspects, R² is benzyl. In other aspects, R² is napthyl. In certain aspects, R² is tolulyl. In other aspects, R² is methoxy. In some aspects, R² is ethoxy. In other aspects R² is propoxy. In some other aspects, R² is butoxy. In some aspects, R² is NR⁴R⁵, wherein R⁴ and R⁵ are independently selected from methyl, ethyl, propyl, butyl, pentyl, hexyl, heptyl, octyl, and nonyl. In certain aspects, R² is dimethylamino or diethylamino.

In certain aspects, R³ is methyl. In other aspects, R³ is ethyl. In some aspects, R³ is propyl (e.g., n-propyl, i-propyl). In other aspects R³ is butyl (e.g., n-butyl, i-butyl). In some other aspects, R³ is pentyl (n-pentyl, i-pentyl , cyclopentyl). In other aspects, R³ is hexyl (n-hexyl, cyclohexyl). In certain aspects, R³ is octyl. In other aspects, R³ is nonyl. In yet others, R³ is dodecyl.

In some aspects of the above formula, R³ is phenyl, benzyl, or napthyl. In other aspects R³ is phenyl. In some other aspects, R³ is benzyl. In other aspects, R³ is napthyl. In certain aspects, R³ is tolulyl. In other aspects, R³ is methoxy. In some aspects, R³ is ethoxy. In other aspects R³ is propoxy. In some other aspects, R³ is butoxy. In some aspects, R³ is NR⁴R⁵, wherein R⁴ and R⁵ are independently selected from methyl, ethyl, propyl, butyl, pentyl, hexyl, heptyl, octyl, and nonyl. In certain aspects, R³ is dimethylamino or diethylamino.

In some aspects, the halogen is selected from F, Cl, Br, or I.

In some aspects, the ligand has the following structure:

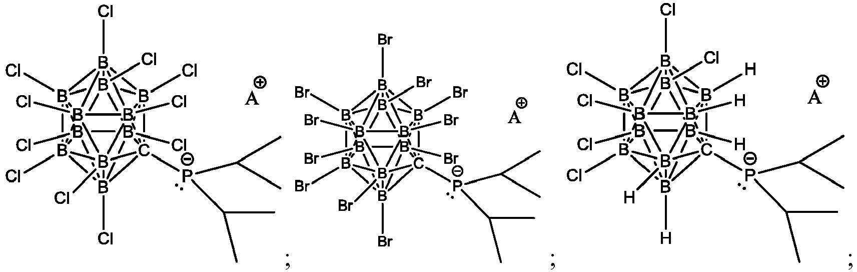

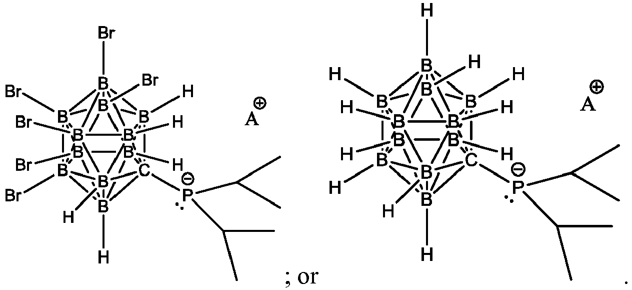

In some other aspects, the present application provides a zwitterionic complex having the following structure: 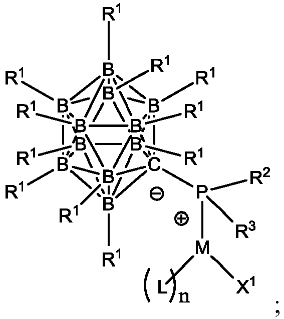 R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. X¹ is selected from H, alkyl, or aryl. L is neutral ligand. Subscript n is an integer selected from 1, 2, 3, 4, 5, or 6. M is a transition metal.

In certain aspect set forth herein, n is 1. In certain other aspect set forth herein, n is 2. In some aspect set forth herein, n is 3. In certain aspect set forth herein, n is 4. In certain aspect set forth herein, n is 5. In certain aspect set forth herein, n is 6.

In some aspects, M is selected from Fe, Ru, Os, Co, Rh, Ir, Ni, Pd, Pt, Cu, Ag, or Au. In other aspects, M is selected from Fe, Ru, Co, Ni, or Pd.

In some aspects of the above formula, R¹ is H. In other aspects R¹ is F. In some other aspects, R¹ is Cl. In other aspects, R¹ is Br. In certain aspects, R¹ is methyl. In other aspects, R¹ is ethyl. In some aspects, R¹ is propyl (e.g., n-propyl, i-propyl). In other aspects R¹ is butyl (e.g., n-butyl, i-butyl). In some other aspects, R¹ is pentyl (n-pentyl, i-pentyl , cyclopentyl). In other aspects, R¹ is hexyl (n-hexyl, cyclohexyl). In certain aspects, R¹ is octyl. In other aspects, R¹ is nonyl. In yet others, R¹ is dodecyl.

In some aspects of the above formula, R¹ is phenyl, benzyl, or napthyl. In other aspects R¹ is phenyl. In some other aspects, R¹ is benzyl. In other aspects, R¹ is napthyl. In certain aspects, R¹ is tolulyl. In other aspects, R¹ is methoxy. In some aspects, R¹ is ethoxy. In other aspects R¹ is propoxy. In some other aspects, R¹ is butoxy. In other aspects, R¹ is trimethylsilyl. In certain aspects, R¹ is triethylsilyl.

In certain aspects, R² is methyl. In other aspects, R² is ethyl. In some aspects, R² is propyl (e.g., n-propyl, i-propyl). In other aspects R² is butyl (e.g., n-butyl, i-butyl). In some other aspects, R² is pentyl (n-pentyl, i-pentyl , cyclopentyl). In other aspects, R² is hexyl (n-hexyl, cyclohexyl). In certain aspects, R² is octyl. In other aspects, R² is nonyl. In yet others, R² is dodecyl.

In some aspects of the above formula, R² is phenyl, benzyl, or napthyl. In other aspects R² is phenyl. In some other aspects, R² is benzyl. In other aspects, R² is napthyl. In certain aspects, R² is tolulyl. In other aspects, R² is methoxy. In some aspects, R² is ethoxy. In other aspects R² is propoxy. In some other aspects, R² is butoxy. In some aspects, R² is NR⁴R⁵, wherein R⁴ and R⁵ are independently selected from methyl, ethyl, propyl, butyl, pentyl, hexyl, heptyl, octyl, and nonyl. In certain aspects, R² is dimethylamino or diethylamino.

In certain aspects, R³ is methyl. In other aspects, R³ is ethyl. In some aspects, R³ is propyl (e.g., n-propyl, i-propyl). In other aspects R³ is butyl (e.g., n-butyl, i-butyl). In some other aspects, R³ is pentyl (n-pentyl, i-pentyl , cyclopentyl). In other aspects, R³ is hexyl (n-hexyl, cyclohexyl). In certain aspects, R³ is octyl. In other aspects, R³ is nonyl. In yet others, R³ is dodecyl.

In some aspects of the above formula, R³ is phenyl, benzyl, or napthyl. In other aspects R³ is phenyl. In some other aspects, R³ is benzyl. In other aspects, R³ is napthyl. In certain aspects, R³ is tolulyl. In other aspects, R³ is methoxy. In some aspects, R³ is ethoxy. In other aspects R³ is propoxy. In some other aspects, R³ is butoxy. In some aspects, R³ is NR⁴R⁵, wherein R⁴ and R⁵ are independently selected from methyl, ethyl, propyl, butyl, pentyl, hexyl, heptyl, octyl, and nonyl. In certain aspects, R³ is dimethylamino or diethylamino.

In some aspects, the present application provides a zwitterionic complex having the following structure:

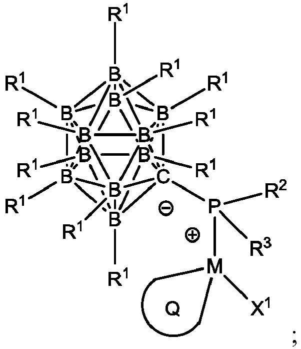

R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. X¹ is selected from the group consisting of H, alkyl, or aryl. Q is a chelating ligand; and M is a transition metal. The complex may further comprising 1 to 2 additional Q chelating ligands bonded to M. In any aspect, M is selected from Fe, Ru, Os, Co, Rh, Ir, Ni, Pd, Pt, Cu, Ag, or Au. In some aspects, M is selected from Fe, Ru, Co, Ni, or Pd.

In some aspects, the present application provides an anionic complex having the following structure:

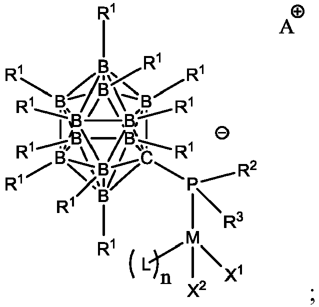

R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. X¹ is selected from H, alkyl, or aryl. X² is an anion selected from H, halide, alkyl, or aryl. A is a cation. L is neutral ligand. Subscript n is an integer selected from 1, 2, 3, 4, 5, or 6. M is a transition metal. In some aspects, M is selected from the group consisting of Fe, Ru, Os, Co, Rh, Ir, Ni, Pd, Pt, Cu, Ag, or Au. In other aspects, M is selected from the group consisting of Fe, Ru, Co, Ni, or Pd.

In some aspects, the present application provides an anionic complex having the following structure:

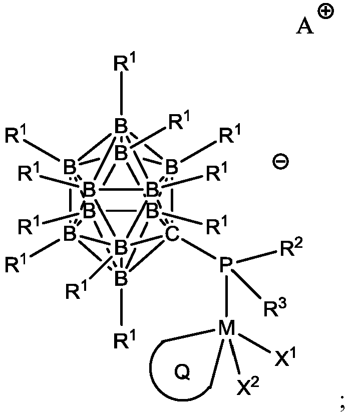

R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. X¹ is selected from H, alkyl, or aryl. X² is an anion selected from H, halide, alkyl, or aryl. Q is a chelating ligand. A is a cation. M is a transition metal. The complex may further comprise 1 to 2 additional Q ligands bonded to M. A is cation. For example, A may be selected from Li, Na, K, Cs, HN(alkyl)₃, or N(alkyl)₄. M is selected from the group consisting of Fe, Ru, Os, Co, Rh, Ir, Ni, Pd, Pt, Cu, Ag, or Au. M is selected from the group consisting of Fe, Ru, Co, Ni, or Pd.

In some aspects, the present application provides a zwitterionic complex having the following structure:

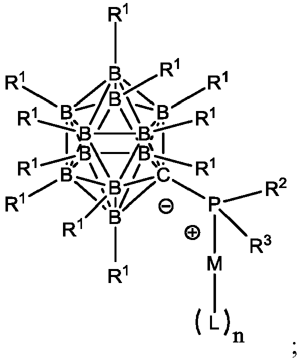

R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. L is neutral ligand. Subscript n is an integer selected from 1, 2, 3, 4, 5, or 6. M is a transition metal. In some aspects, M is selected from Fe, Ru, Os, Co, Rh, Ir, Ni, Pd, Pt, Cu, Ag, or Au. In other aspects, M is selected from Fe, Ru, Co, Rh, Ir, Ni, Pd, Pt, Cu, Ag, or Au.

In some preferred aspects, L is Cl. In other aspects, L is tetrahydrothiophene.

In some aspects, the present application provides a zwitterionic complex having the following structure:

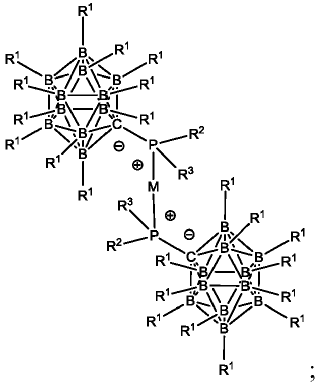

R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. M is a transition metal. In some aspects, M is selected from Fe, Ru, Os, Co, Rh, Ir, Ni, Pd, Pt, Cu, Ag, or Au. In other aspects, M is selected from Ni, Pd, or Cu. In some of these aspects, M is in either a +2 or +4 oxidation state.

In some aspects, the present application provides an anionic complex having the following structure:

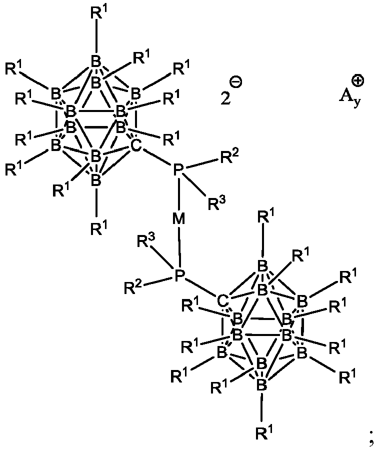

R¹ is selected from H, halogen, alkyl, aryl, silyl, or alkoxy. R² and R³ are each independently selected from alkyl, aryl, alkoxy, or NR⁴R⁵. R⁴ and R⁵ are each independently selected from alkyl, aryl, or alkoxy. A is a monopositively charged cation or a dipositively charged cation. Y is 1 or 2. M is a transition metal. In some aspects, M is selected from Fe, Ru, Os, Co, Rh, Ir, Ni, Pd, Pt, Cu, Ag, or Au. In other aspects, M is selected from Ni, Pd, or Cu. In certain aspects, M is in either a +2 or +4 oxidation state.

In some aspects, the present application provides a complex having the following structure:

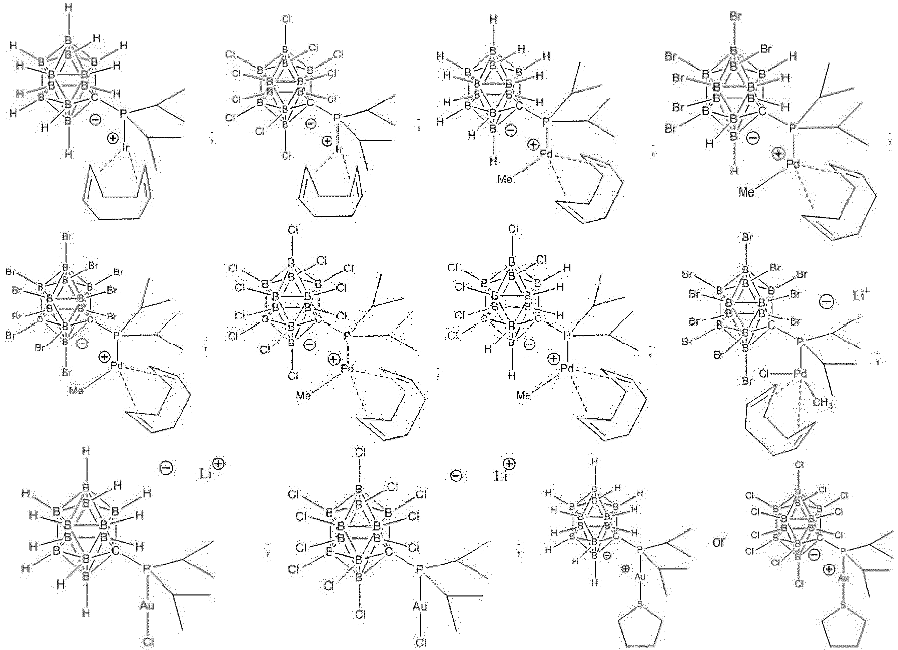

In some aspects of the above formula, R¹ is H. In other aspects R¹ is F. In some other aspects, R¹ is Cl. In other aspects, R¹ is Br. In certain aspects, R¹ is methyl. In other aspects, R¹ is ethyl. In some aspects, R¹ is propyl (e.g., n-propyl, i-propyl). In other aspects R¹ is butyl (e.g., n-butyl, i-butyl). In some other aspects, R¹ is pentyl (n-pentyl, i-pentyl , cyclopentyl). In other aspects, R¹ is hexyl (n-hexyl, cyclohexyl). In certain aspects, R¹ is octyl. In other aspects, R¹ is nonyl. In yet others, R¹ is dodecyl.

In any of the above aspects, R¹ is phenyl, benzyl, or napthyl. In other aspects R¹ is phenyl. In some other aspects, R¹ is benzyl. In other aspects, R¹ is napthyl. In certain aspects, R¹ is tolulyl. In other aspects, R¹ is methoxy. In some aspects, R¹ is ethoxy. In other aspects R¹ is propoxy. In some other aspects, R¹ is butoxy. In other aspects, R¹ is trimethylsilyl. In certain aspects, R¹ is triethylsilyl.

In certain of the above aspects, R² is methyl. In other aspects, R² is ethyl. In some aspects, R² is propyl (e.g., n-propyl, i-propyl). In other aspects R² is butyl (*e.g*., n-butyl, i-butyl). In some other aspects, R² is pentyl (n-pentyl, i-pentyl , cyclopentyl). In other aspects, R² is hexyl (n-hexyl, cyclohexyl). In certain aspects, R² is octyl. In other aspects, R² is nonyl. In yet others, R² is dodecyl.

In some of the above aspects of the above formula, R² is phenyl, benzyl, or napthyl. In other aspects R² is phenyl. In some other aspects, R² is benzyl. In other aspects, R² is napthyl. In certain aspects, R² is tolulyl. In other aspects, R² is methoxy. In some aspects, R² is ethoxy. In other aspects R² is propoxy. In some other aspects, R² is butoxy. In some aspects, R² is NR⁴R⁵, wherein R⁴ and R⁵ are independently selected from methyl, ethyl, propyl, butyl, pentyl, hexyl, heptyl, octyl, and nonyl. In certain aspects, R² is dimethylamino or diethylamino.

In certain of the above aspects, R³ is methyl. In other aspects, R³ is ethyl. In some aspects, R³ is propyl (e.g., n-propyl, i-propyl). In other aspects R³ is butyl (*e.g*., n-butyl, i-butyl). In some other aspects, R³ is pentyl (n-pentyl, i-pentyl , cyclopentyl). In other aspects, R³ is hexyl (n-hexyl, cyclohexyl). In certain aspects, R³ is octyl. In other aspects, R³ is nonyl. In yet others, R³ is dodecyl.

In some of the above aspects of the above formula, R³ is phenyl, benzyl, or napthyl. In other aspects R³ is phenyl. In some other aspects, R³ is benzyl. In other aspects, R³ is napthyl. In certain aspects, R³ is tolulyl. In other aspects, R³ is methoxy. In some aspects, R³ is ethoxy. In other aspects R³ is propoxy. In some other aspects, R³ is butoxy. In some aspects, R³ is NR⁴R⁵, wherein R⁴ and R⁵ are independently selected from methyl, ethyl, propyl, butyl, pentyl, hexyl, heptyl, octyl, and nonyl. In certain aspects, R³ is dimethylamino or diethylamino.

In some of the above aspects, L is a neutral ligand selected from cyclooctadiene.

In certain preferred aspects of the above, R² and R³ are isopropyl.

Certain Preferred Substituents include the following.

In some of the above aspects, preferred R¹ groups include, but are not limited to carborane H, F, Cl, Br, I, benzene, methyl, ethyl, n-propyl, n-butyl, methoxy,eEthoxy, n-propoxy , n-butyloxy, or trimethylsiloxy.

In some of the above aspects, preferred R² groups include benzene, naphthyl, Mesityl, 2,6-diisopropylphenyl, Tolyl, or -C₆F₅. In some of the above aspects, preferred R² groups include methyl, ethyl, propyl, butyl, isopropyl, t-butyl, adamantly, cyclohexyl, or cyclopentyl. In some of the above aspects, preferred R² groups include methoxy, ethoxy, propoxy , butyloxy, isopropoxy, t-butoxy, phenoxy, or mesityloxy. In some of the above aspects, preferred R² groups include Trimethylsiloxy, triethyl siloxy, or tripropyl siloxy.

In some of the above aspects, preferred R⁴ and R⁵ include H. In other preferred aspects, R⁴ and R⁵, independently, include benzene, naphthyl, Mesityl, 2,6-diisopropylphenyl, Tolyl, or -C₆F₅. In other preferred aspects, R⁴ and R⁵ include, independently, methyl, ethyl, propyl, butyl, isopropyl, t-butyl, adamantly, cyclohexyl, or cyclopentyl. In other preferred aspects, R⁴ and R⁵ include trimethylsilyl, triethylsilyl, or tributylsilyl.

In some preferred aspects, R² or R³ are independently selected from NR⁴R⁵, wherein R⁴ and R⁵ are H, benzene, naphthyl, Mesityl, 2,6-diisopropylphenyl, Tolyl, -C₆F₅, methyl, ethyl, propyl, butyl, isopropyl, t-Butyl, adamantly, cyclohexyl, cyclopentyl, trimethylsilyl, triethylsilyl, or tributylsilyl.

In some preferred aspects, X¹ and X² are independently selected from NR⁴R⁵, wherein NR⁴R⁵ is imido. In some aspects, R⁴ and R⁵ are N-Alkyl, NAryl, Sulfido S-R, or Phosphido P-R.

In some of the above aspects, preferred R³ groups include benzene, naphthyl, Mesityl, 2,6-diisopropylphenyl, Tolyl, or -C₆F₅. In some of the above aspects, preferred R³ groups include methyl, ethyl, propyl, butyl, isopropyl, t-butyl, adamantly, cyclohexyl, or cyclopentyl. In some of the above aspects, preferred R³ groups include methoxy, ethoxy, propoxy , butyloxy, isopropoxy, t-butoxy, phenoxy, or mesityloxy. In some of the above aspects, preferred R³ groups include Trimethylsiloxy, triethyl siloxy, or tripropyl siloxy.

In other preferred aspects, L is a neutral ligand. In some aspects, L is a solvent molecule selected from dimethoxy ethane, dioxane, pyridine, tetrahydrofuran, diethyl ether, benzene, toluene, acetonitrile, methylene chloride, chloroform, dimethyl formamide, or acetone. In other preferred aspects, L is a phosphine (PR₃) containing three alkyl or aryl groups, A phosphite (P(OR)₃), P(NR₂)₃, a carbene, N-heterocyclic carbene, an alkylidene carbene, a benzylidene carbene, an olefin, an alkyne, a diene, an enone, a polyene, a sulfide (SR₂), an alcohol, a ketone, or an ester.

In some preferred aspects, A is selected frm Li⁺, Na⁺, K⁺, Cs⁺, Mg²⁺, Ca²⁺, Zn²⁺, NH₄⁺, HN(Alkyl)₃⁺, HN(Me)₃⁺, HN(Ethyl)₃⁺,HN(Butyl)₄⁺, HN(Aryl)₃⁺, H₂N(Alkyl)₂⁺, H₃NAlkyl⁺, H₃NAryl⁺, a transition metal cation, a main group cation, an alkaline earth cation, or a polyatomic cation.

In some preferred aspects, X¹ and X² are independently selected from H, F, Cl, Br, I. In some preferred aspects, X¹ and X² are independently selected from methyl, ethyl, propyl, butyl, isopropyl, t-Butyl, adamantly, cyclohexyl, cyclopentyl, neopentyl,-CH₂SiMe₃, benzyl.

In some preferred aspects, X¹ and X² are independently selected from -C₆H₅, naphthyl, Mesityl, or Tolyl.

In some preferred aspects, X¹ and X² are independently selected from methoxy, ethoxy, propoxy , butyloxy, isopropoxy, t-butoxy, phenoxy, or mesityloxy.

In some preferred aspects, X¹ and X² are independently selected from Trimethylsiloxy, triethyl siloxy, or tripropyl siloxy).

In some preferred aspects, X¹ and X² are independently selected from NR⁴R⁵, wherein R⁴ and R⁵ are H, benzene, naphthyl, Mesityl, 2,6-diisopropylphenyl, Tolyl, -C₆F₅, methyl, ethyl, propyl, butyl, isopropyl, t-Butyl, adamantly, cyclohexyl, cyclopentyl, trimethylsilyl, triethylsilyl, or tributylsilyl.

In some preferred aspects, X¹ and X² are independently selected from NR⁴R⁵, wherein NR⁴R⁵ is imido. In some aspects, R⁴ and R⁵ are N-Alkyl, NAryl, Sulfido S-R, or Phosphido P-R.

### METHOD OF MAKING COMPOUNDS AND COMPLEXES

In some aspects, the present disclosure provides methods of making ligands having the following general formula: 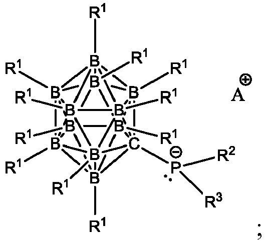 In these aspects, R¹ is selected from the group consisting of H, halide, alkyl, aryl, silyl, and alkoxy. R² and R³ are each independently selected from the group consisting of alkyl, aryl, alkoxy, and NR⁴R⁵. In these aspects, R⁴ and R⁵ are each independently selected from the group consisting of alkyl, aryl, and alkoxy. In any aspect set forth herein, halide includes a member selected from the group consisting of fluoride, chloride, bromide, and iodide. A⁺ represents a counter-cation. In some aspects, A is selected from the group consisting of Li⁺, Na⁺, K⁺, Cs⁺, HN(alkyl)₃⁺, and N(alkyl)₄⁺. Other suitable cations may include, but are not limited to a member selected from the group consisting of Be²⁺, Mg²⁺, Ca²⁺, Sr²⁺, and Ba²⁺.

In some aspects, the methods of making the ligands used herein include providing a solution of a C-H carba-*closo*-dodecaborate anion, having the following formula, in THF:

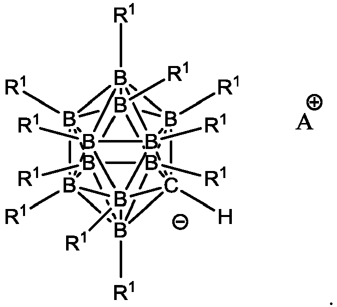

In certain aspects, the methods further include treating the anion with 1-3 equivalents of a base, wherein the base is n-BuLi (*i.e.,* n-butyl lithium). In some aspects, the methods then include stirring for about three hours. In some aspects, the methods further include concentrating the reaction under high vacuum. In some of these aspects, the methods include washing the resulting residue with dry hexane to prepare a solid. In some aspects, the methods include dissolving the solid in THF or F-C₆H₅ and treating it with 1.1 equivalents of the X-P(R²)(R³) reagent. In some aspects, the methods include stirring the reaction until completion. In certain aspects, completion is determined by ³¹P NMR analysis. In some aspects, the methods include concentrating the mixture to dryness, washing the concentrate with hexane. In some aspects, the methods include including the solid residue (i.e., the phosphine ligand) extracted in a solvent, wherein the solvent includes, but is not limited to CHCl₃, CH₂Cl₂, F-C₆H₅). In some of these aspects, the salt byproducts do not dissolve in the solvent. In some aspects, the methods include filtering the mixture and drying the solution to afford the desired phosphine in about 95-100% yield.

In some aspects, the present disclosure provides methods illustrated by the following reaction scheme:

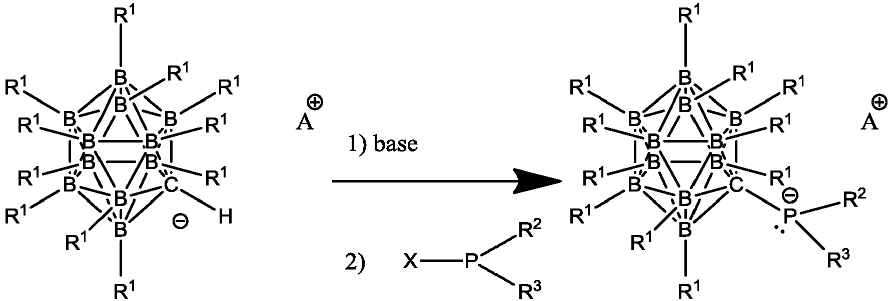

In any of the aspects set forth herein, X refers to a leaving group. In some of these aspects, the leaving group is selected from the group consisting of Cl, Br, and I.

In some aspects, the present diclosure provides methods of making ligands that include, but are not limited to the following synthesis. A solution of the C-H carba-closo-dodecaborate anion in THF is treated with 1-3 equivalents of a base (preferably *n*-BuLi) and stirred for 3 hours and subsequently concentrated under high vacuum. The residue is thoroughly washed with dry hexane and the solid is subsequently dissolved in THF or F-C₆H₅ and treated with 1.1 equivalents of the desired X-P(R²)(R³) reagent and the reaction is stirred until completion is determined by ³¹P NMR analysis. The mixture is subsequently concentrated to dryness, washed with hexane, and the solid residue containing the phosphine ligand extracted with a solvent (e.g. CHC13, CH₂Cl₂, F-C₆H₅) where the salt byproducts do not dissolve. The mixture is filtered and the solution is concentrated to dryness to afford the desired phosphine in 95-100% yield.

### CATALYTIC REACTIONS

The instant application sets forth methods of catalyzing chemical reactions, wherein the methods include contacting a complex, set forth herein, with suitable reagents for the chemical reaction to occur. In some aspects, the chemical reaction catalyzed is olefin polymerization. In some other aspects, the chemical reaction catalyzed is hydroamination. In some other aspects, the chemical reaction catalyzed is cross coupling. In some other aspects, the chemical reaction catalyzed is hydrogenation.

The literature describing Au-based catalysts includes a) H. Schmidbaur, et al., B: J. Chem. Sci. 2011, 66, 329-350; Synthesis (Eds.: A.S.K. Hashmi, F. D. Toste), Wiley-VCH, Weinheim, 2012; A. Gómez-Suárez, et al., Angew. Chem. 2012, 124, 8278-8281; Angew. Chem. Int. Ed. 2012, 51, 8156-8159; C. C. J. Loh, et al., Chem. Eur. J. 2012, 18, 10212-10225; D. Garayalde, et al., ACS Catal. 2012, 2, 1462-1479; D. Wang, et al., J. Am. Chem. Soc. 2012, 134, 9012-9019; f) B.-L. Lu, L. Dai, M. Shi, Chem. Soc. Rev. 2012, 41, 3318-3339; L.-P. Liu, et al., Chem. Soc. Rev. 2012, 41, 3129-3139; M. Rudolph, et al., Chem. Soc. Rev. 2012, 41, 2448-2462; C. D. Pina et al., Chem. Soc. Rev. 2012, 41, 350-369; M. Rudolph, et al., Chem. Commun. 2011, 47, 6536-6544; M. Bandini, Chem. Soc. Rev. 2011, 40, 1358-1367; M. Malacria, , Top. Curr. Chem. 2011, 302, 157-182; A. S. K. Hashmi, Angew. Chem. 2010, 122, 5360-5369; Angew. Chem. Int. Ed. 2010, 49, 5232-5241; D. J. Gorin, et al., Chem. Rev. 2008, 108, 3351-3378.

The literature describing hydroamination reactions includes J. L. Klinkenberg, et al., Angew. Chem. 2011, 123, 88-98; Angew. Chem. Int. Ed. 2011, 50, 86-95; I. Krossing, Angew. Chem. 2011, 123, 11781-11783; Angew. Chem. Int. Ed. 2011, 50, 11576-11578; T. E. Müller, et al., Chem. Rev. 2008, 108, 3795-3892; R. A. Widenhoefer, et al., Eur. J. Org. Chem. 2006, 2006, 4555-4563.

The literature describing gold catalyzed hydroamination of alkynes includes S. Fleischer, et al., Chem. Eur. J. 2012, 18, 9005-9010; E. Alvarado, et al., Chem. Eur. J. 2012, 18, 12112-12121; R. Kinjo, et al., Angew. Chem. 2011, 123, 5674-5677; Angew. Chem. Int. Ed. 2011, 50, 5560-5563; K. L. Butler, et al., Angew. Chem. 2012, 124, 5265-5268; Angew. Chem. Int. Ed. 2012, 51, 5175-5178; K. D. Hesp, et al., J. Am. Chem. Soc. 2010, 132, 18026-18029; A. Leyva-Pérez, et al., J. Org. Chem. 2010, 75, 7769-7780; V. Lavallo, et al., Angew. Chem. 2008, 120, 5302-5306; Angew. Chem. Int. Ed. 2008, 47, 5224-5228; E. Mizushima, et al., Org. Lett. 2003, 5, 3349-3352)

In some aspects, the instant application provides methods of catalyzing olefin polymerization reactions comprising contacting a complex described herein with suitable olefin polymerization reagents.

In some aspects, the instant application provides methods of catalyzing hydroaddition reactions comprising contacting a complex described herein with suitable hydro addition reagents.

In some aspects, the instant application provides methods of catalyzing hydroamination reactions comprising contacting a complex described herein with suitable hydroamination reagents.

In some aspects, the instant application provides methods of catalyzing cross coupling reactions contacting a complex described herein with suitable cross coupling reagents.

In some aspects, the instant application provides methods of catalyzing olefin metathesis reactions comprising contacting a complex described herein with suitable olefin metathesis reagents.

In some aspects, the instant application provides methods of catalyzing hydroformylation reactions comprising contacting a complex described herein with suitable hydroformylation reagents.

In some aspects, the instant application provides methods of catalyzing hydrogenation reactions comprising contacting a complex described herein with suitable hydrogenation reagents.

In some aspects, the instant application provides methods of catalyzing hydroaminomethylation reactions comprising contacting a complex described herein with suitable hydroaminomethylation reagents.

### EXAMPLES

### Example 1 - Synthesis of Ligands

A solution of the C-H carba-*closo*-dodecaborate anion in THF was treated with 1-3 equivalents of a base (most preferably n-BuLi) and stirred for 3 hours and subsequently concentrated under high vacuum. The residue was thoroughly washed with dry hexane and the solid was subsequently dissolved in THF or F-C₆H₅ and treated with 1.1 equivalents of the desired X-P(R2)(R3) reagent and the reaction was stirred until completion was determined by ³¹P NMR analysis. The mixture was subsequently concentrated to dryness, washed with hexane, and the solid residue containing the phosphine ligand extracted with a solvent (e.g. CHC13, CH2C12, F-C6H5) where the salt byproducts do not dissolve. The mixture was filtered and the solution was concentrated to dryness to afford the desired phosphine in 95-100% yield.

Ligands synthesized by this method include the following examples:
iPr₂PCB₁₁Cl₁₁⁻Li⁺ (x 4 THF); ³¹P-(¹H-dec) NMR (121 MHz, CD₂Cl₂, 25 °C) δ =77.3 ppm
iPr₂PCB₁₁H₅Cl₆⁻Li⁺ (x 4 THF); ³¹P-(¹H-dec) NMR (121 MHz, CD₂Cl₂, 25 °C) δ =86.6 ppm
iPf₂PCB₁₁H₁₁⁻Li⁺ (x 4 THF); ³¹P-(¹H-dec) NMR (121 MHz, CD₂Cl₂, 25 °C) δ = 45.4 ppm
iPr₂PCB₁₁H₁₁⁻N(Butyl₄)⁺; ³¹P-(¹H-dec) NMR (121 MHz, CD₂Cl₂, 25 °C) δ =45.6 ppm
iPr₂PCB₁₁H₁₁⁻N(Me₄)⁺; ³¹P-(¹H-dec) NMR (121 MHz, CD₂Cl₂, 25 °C) δ =45.6 ppm
iPr₂PCB₁₁Br₁₁⁻Li⁺ (x 4 THF); ³¹P-(¹H-dec) NMR (121 MHz, CD₂Cl₂, 25 °C) δ =100.2 ppm
iPr₂PCB₁₁H₅Br₆⁻Li⁺ (x 4 THF); ³¹P-(¹H-dec) NMR (121 MHz, CD₂Cl₂, 25 °C) δ =95.0 ppm
Ph₂PCB₁₁Br₁₁⁻Li⁺ (x 4 THF); ³¹P-(¹H-dec) NMR (121 MHz, CD₂Cl₂, 25 °C) δ = 106.6 ppm
Ph₂PCB₁₁Cl₁₁⁻Li⁺ (x 4 THF); ³¹P-(¹H-dec) NMR (121 MHz, CD₂Cl₂, 25 °C) δ = 87.8 ppm
Et₂PCB₁₁Br₁₁⁻Li⁺ (x 4 THF); ³¹P-(¹H-dec) NMR (121 MHz, CD₂Cl₂, 25 °C) δ =99.7 ppm

### Example 2 - Synthesis of a Catalyst

This catalyst is suitable for use in, for example, olefin polymerization

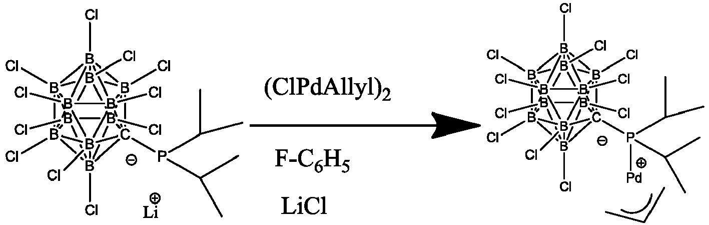

A solution of the phosphine ligand iPr₂PCB₁₁Cl₁₁⁻ Li⁺ (x 4 THF) (1 mmol, 933.421 mg) was dissolved in 20 mL FC₆H₅ and reacted with a solution of (ClPdAllyl)₂ (0.5 mmol 182.94 mg) that was dissolved in 10 mL of FC₆H₅. The resulting mixture was stirred and a precipitate of LiCl and the desired complex rapidly formed. After 15 minutes the precipitate was collected and washed with 10 mL of FC₆H₅. The desired complex was extracted from the LiCl precipitate with 100 mL of CH₂Cl₂, filtered, and the remaining solid LiCl discarded. The CH₂Cl₂ solution was concentrated under high vacuum to afford the desired complex in 95% yield (0.95 mmol, 746.3 mg).
¹H NMR (300 MHz, CD₂Cl₂, 25 °C): δ=5.78 (m, 1H), 4.92 (s-br, 2H), 3.74 (s-br, 2H), 3.53 (m, 2H), 1.52 (m, 12H); ³¹P-(¹H-dec) NMR (121 MHz, CD₂Cl₂, 25 °C): δ=117.61; ¹³C NMR (100 MHz, CD₂Cl₂, 25 °C): δ=119.41, 29.85, 27.17, 22.58; ¹¹B-(¹H-dec) NMR (96 MHz, CD₂Cl₂, 25 °C): δ= 1.38, -7.48, -9.60 ppm.

### Reference Example 3 - Olefin Polymerization

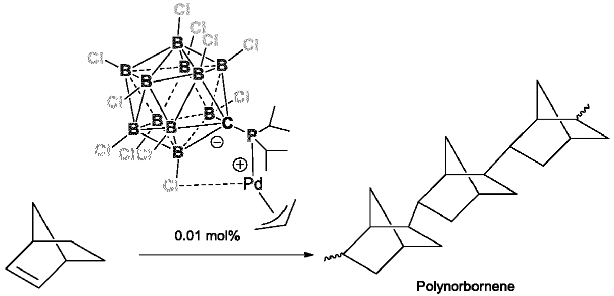

The catalyst above (0.01 mmol) was dissolved in CH₂Cl₂. Subsequent addition of norbornene (1 mmol) monomer and stirring the mixture for 1 hour, afforded polynorbornene in 82% yield. The polymer was isolated by the addition of methanol to the mixture, whereupon the polymer precipitated.

### Example 4 - Synthesis of a Catalyst

This catalyst is suitable for use in, for example, olefin hydrogenation

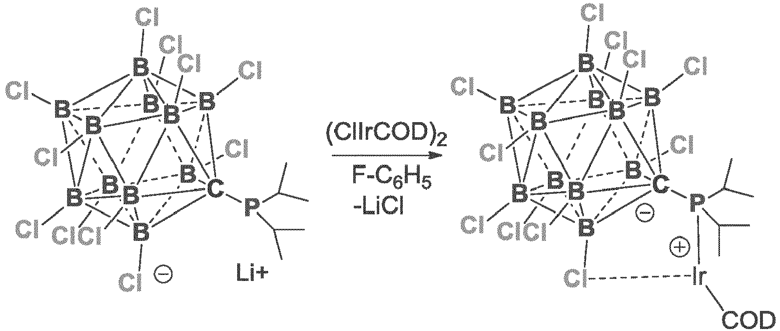

A solution of the phosphine ligand iPr₂PCB₁₁Cl₁₁⁻ Li⁺ (x 4 THF) (1 mmol, 933.421 mg) dissolved in 20 mL FC₆H₅ was reacted with a solution of (ClIr(COD))₂ (0.5 mmol 335.85 mg) dissolved in 40 mL of FC₆H₅. The mixture was stirred for 24 hours and a precipitate of LiCl formed. The solution was filtered and concentrated under high vacuum to afford the desired complex in 100% yield (1.0 mmol, 938.5 mg). Structure was confirmed by single crystal diffraction study and NMR ³¹P-(¹H-dec) NMR (121 MHz, CD₂Cl₂, 25 °C): δ=100.54 ppm

### Reference Example 5 - Olefin Hydrogenation

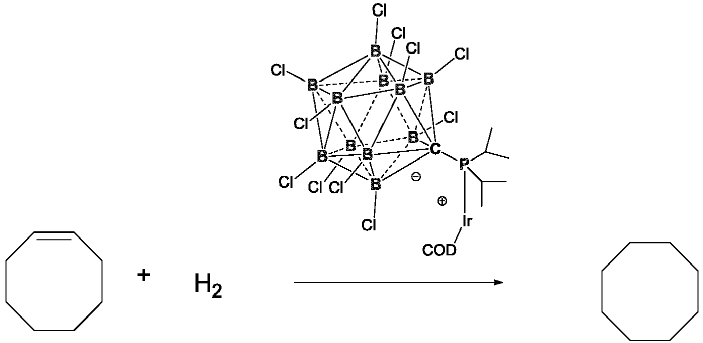

Procedure: The catalyst above (0.1 mmol) was dissolved in a solution of cyclooctene (2 mmol)/5 mL THF. The mixture was exposed to 1.01325 bar (1 atmosphere) of hydrogen gas and heated to 25°C for 30 minutes. ¹H NMR analysis of the mixture revealed complete hydrogenation of cyclooctene to afford cyclooctane in quantitative yield (2 mmol, 100%).

### Reference Example 6 - Synthesis of a Catalyst

This catalyst is suitable for use in, for example, olefin polymerization.

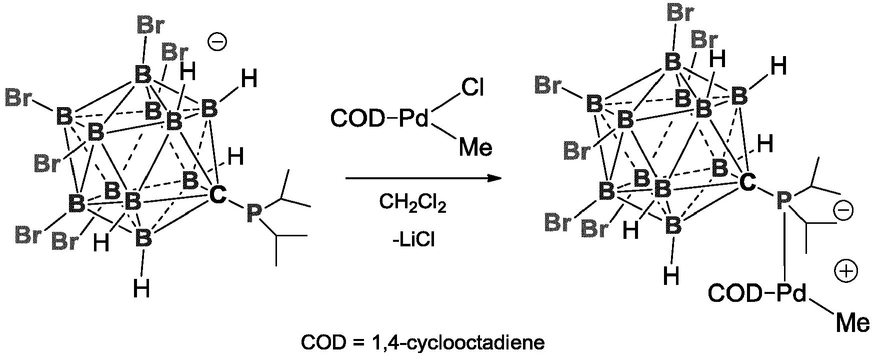

**Synthesis of PdCH₃(COD)iPr₂PCB₁₁H₅Br₆** A solution of iPr₂PCB₁₁H₅Br₆⁻ Li⁺(x 4 THF) (774.78 mg, 1.0 mmol) in THF, was combined with a solution of PdCH₃Cl(COD) (265.1 mg, 1 mmol) in THF and the reaction mixture was allowed to stir for 1 hour. Lithium Chloride was precipitated out by the addition of CHCl₃ and the solution filtered. Volatiles were removed under reduced pressure and washed with hexanes (2 x 2mL). The zwitterionic complex **2** was obtained as a light yellow colored powder (694.82 mg, 98 % yield). ¹H NMR (300 MHz, CD₂Cl₂, 25 °C): δ=5.89 (m, 2H), 5.39 (m, 2H), 2.68 (m, 4H) 2.54 (m, 4H), 2.38 (m, 2H), 1.48 (m, 12H), 1.31 (s, 3H); ³¹P-(¹H-dec) NMR (121 MHz, CD₂Cl₂, 25 °C): δ = 68.32

### Reference Example 7 - Olefin Polymerization

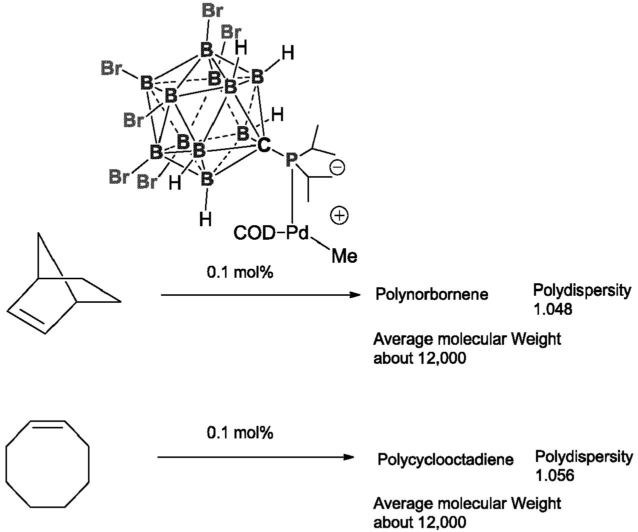

The catalyst above (0.1 mmol) was dissolved in CH₂Cl₂. Subsequent addition of monomer (1 mmol) and stirring the mixture for 1 hour, afforded polynorbornene (98% yield) and Polycyclooctene (93% yield), respectively. The polymer was isolated by the addition of methanol to the mixture, whereupon the polymer precipitated. Analysis of the material by Gel permeation chromatography reveals a very low polydispersity of 1.048 (polynorbornene) and 1.056 (polycyclooctene) and an average molecular weight of approximately 12,000. These data indicate that the catalyst is quite active.

### Reference Example 8 - Synthesis of a Catalyst

This catalyst is suitable for use in, for example, olefin polymerization

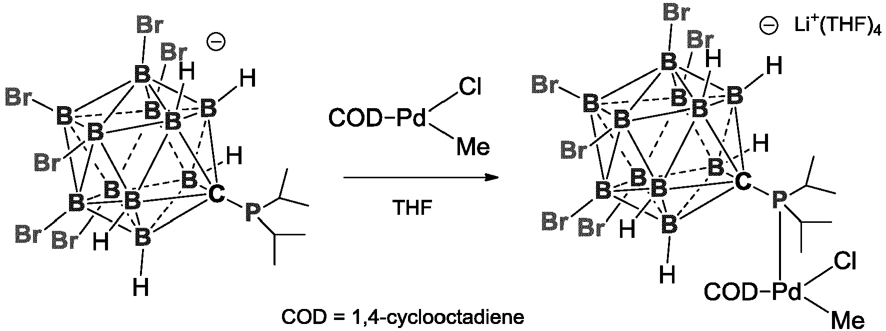

### Synthesis of PdCH₃Cl(COD)iPr₂PCB₁₁H₅Br₆

A solution of iPr₂PCB₁₁H₅Br₆⁻ Li⁺ (x 4 THF) (1 (774.78 mg, 1.0 mmol) in THF, was combined with a solution of PdCH₃Cl(COD) (265.1 mg, 1 mmol) in THF and the reaction mixture was allowed to stir for 1 hour. Volatiles were removed under reduced pressure and washed with hexanes (2 x 2mL) to afford the complex in quantitative yield (1 mmol, 1.039 g). ³¹P-(¹H-dec) NMR (121 MHz, THF, 25 °C): δ = 71.30 ppm.

### Reference Example 9 - Olefin Polymerization

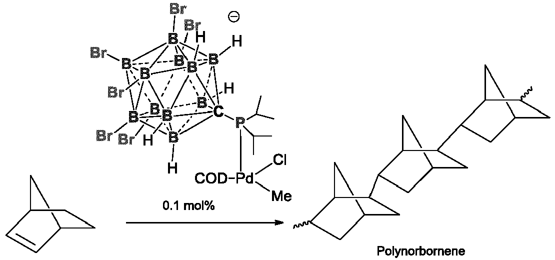

The catalyst above (0.1 mmol) was dissolved in THF. Subsequent addition of norbornene (1 mmol) monomer and stirring the mixture for 4 hours, afforded polynorbornene in 95% yield. The polymer was isolated by the addition of methanol to the mixture, whereupon the polymer precipitated.

### Reference Example 10 - Synthesis of a Catalyst

This catalyst is suitable for use in, for example, Pd-catalyzed cross-coupling.

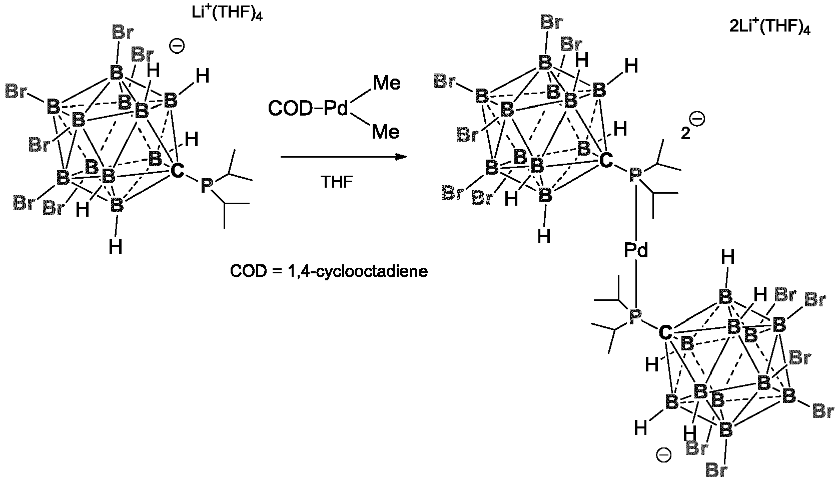

**Synthesis of Pd(iPr₂PCB₁₁H₅Br₆)²⁻ 2Li⁺(x 4 THF)**. A solution of iPr₂PCB₁₁H₅Br₆⁻ Li⁺ (x 4 THF) (774.78 mg, 1.0 mmol) in THF, was combined with a solution of (CH₃)₂Pd (COD) (123.0 mg, 0.5 mmol) in THF and the reaction mixture was allowed to stir for 1 hour. Volatiles were removed under reduced pressure to afford the desired dianionic Pd(0) complex a dark colored (828.0 mg, 100 % yield). The structure was confirmed by a preliminary X-ray diffraction study and NMR. ³¹P-(¹H-dec) NMR (121 MHz, THF, 25 C): δ = 53.41 ppm.

### Reference Example 11 - Pd Cross-coupling (alpha arylation of ketones)

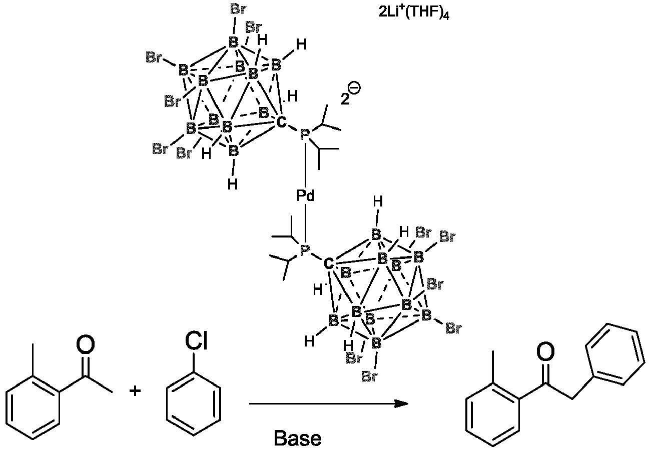

The catalyst above (0.1 mmol) was dissolved in THF. Subsequent addition of the ketone (1 mmol), chlorobenzene (1 mmol) and NaOtBu (1.05 mmol) followed by heating at 80°C for 6 hours, afforded the desired alpha-arylated ketone in 89% yield as indicated by ¹H NMR spectroscopy.

### Reference Example 12 - Synthesis of a Catalyst

This catalyst is suitable for use in, for example, olefin hydrogenation

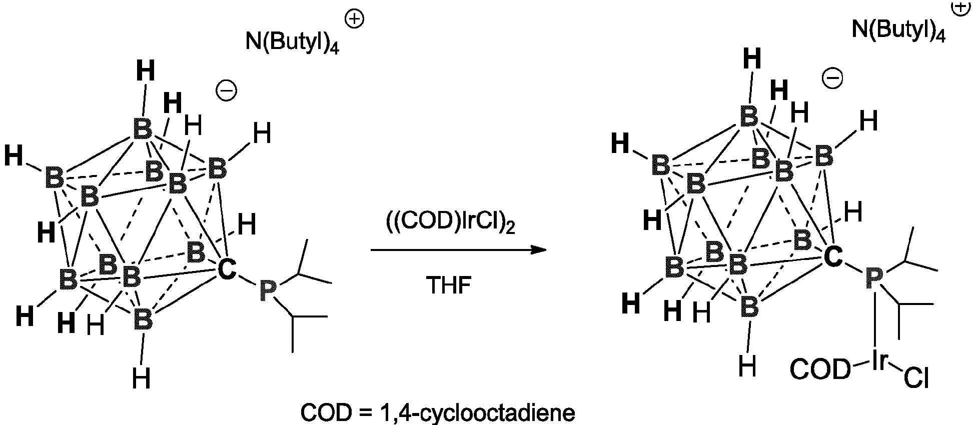

Synthesis of anionic ClIr(COD)[P(C₃H₇)₂CB₁₁H₁₁]: P(C₃H₇)₂(CB₁₁H₁₁)⁻ N(Butyl)₄⁺ (501.6 mg, 1 mmol) in benzene (5 mL) was added to a benzene (5 mL) solution of (Ir(COD)Cl)₂ (335.8 mg, 0.5 mmol) and allowed to stir overnight. The mixture was concentrated under vacuum and washed with hexanes (2 x 5mL), to afford the desired anionic complex as a yellow-orange solid in 100% yield (837.4 mg, 1mmol mmol). ³¹P NMR (242 MHz, C6D6, 25 °C): δ=37.46 ppm.

### Reference Example 13 - Olefin Hydrogenation

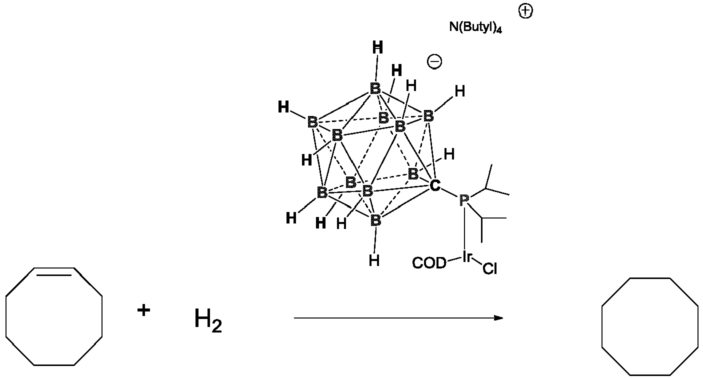

Procedure: The catalyst above (0.1 mmol) was dissolved in a solution of cyclooctene (1 mmol)/5 mL THF. The mixture was exposed to 1.01325 bar (1 atmosphere) of hydrogen gas and heated to 50°C for 2 hours. ¹H NMR analysis of the mixture revealed complete hydrogenation of cyclooctene to afford cyclooctane in quantitative yield (1 mmol, 100%).

### Reference Example 14 - Hydrogenation

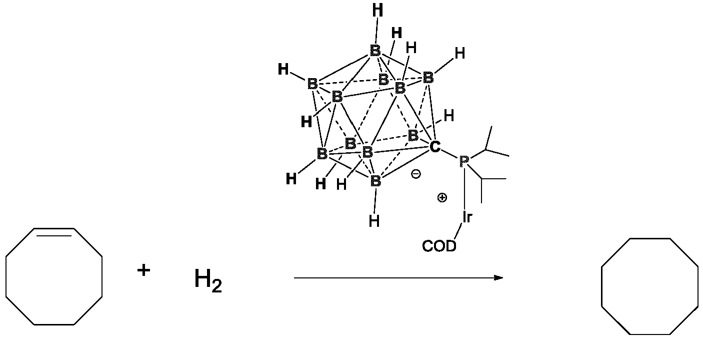

Procedure: The catalyst above (0.1 mmol) was dissolved in a solution of cyclooctene (2 mmol)/5 mL THF. The mixture was exposed to 1.01325 bar (1 atmosphere) of hydrogen gas and heated to 40°C for 1 hour. ¹H NMR analysis of the mixture revealed complete hydrogenation of cyclooctene to afford cyclooctane in quantitative yield (2 mmol, 100%).

### Example 15 - Synthetic Procedure

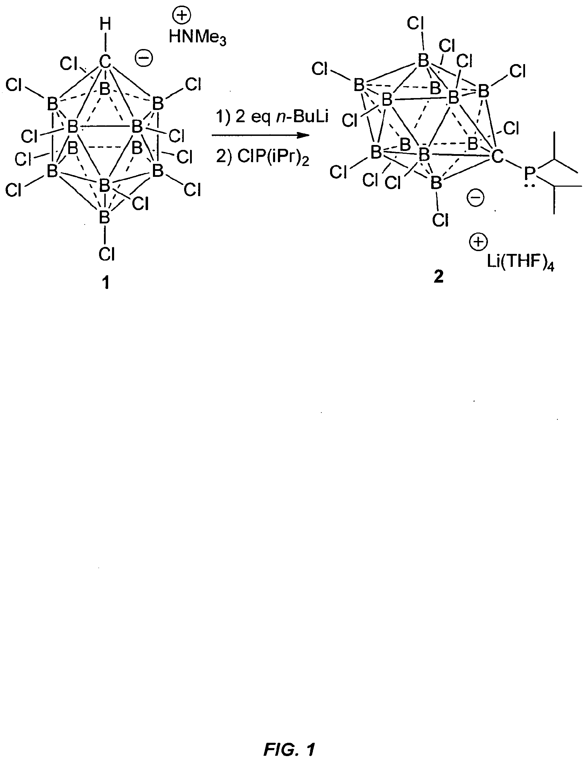

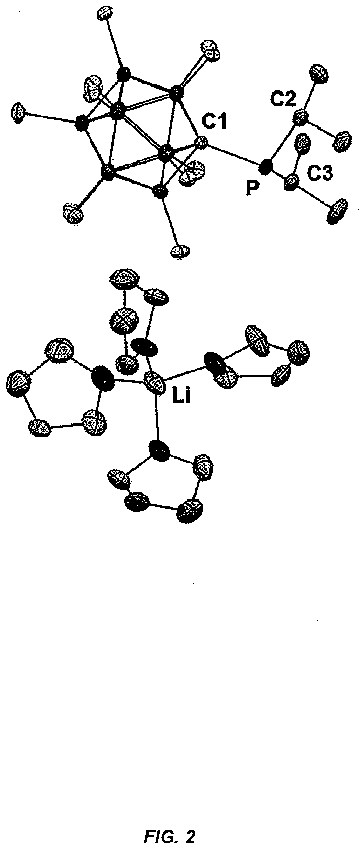

This Example shows a synthesis of a phosphine bearing the CB₁₁Cl₁₁⁻ moiety, (iPr)₂P(CB₁₁Cl₁₁)⁻Li⁺. This group is advantageous because it is very large and the intermediate steric bulk of the isopropyl groups allow the phosphorus lone pair to be accessible for coordination. The trimethyl ammonium salt of anion 1 with 2 (in Figure 1) was treated with equivalents of *n*-BuLi and subsequent quenched with the dianionic intermediate with ClP(iPr)₂ afforded 2 in 98% yield (Figure 1). Anionic phosphine 2 is very soluble in common polar solvents (CH₂Cl₂, CHCl₃, THF), and is not sensitive to oxygen in solution or the solid state. The ¹H NMR of 2 displays two distinct doublets of doublets for the CH₃ isopropyl groups, indicating hindered rotation about the P-iPr bonds. A single crystal X-ray diffraction study shows a sterically congested environment around the pyramidalized (sum of CPC angles = 325.1°) phosphorus center (Figure 2). The phosphorus bond to the carborane hypercarbon (P-C1 = 1.9376(16)Å) is significantly longer than the P-iPr bonds (P-C2 = 1.8679(19), P-C3 = 1.8636(18)Å). The lithium countercation of 2 is coordinated by four THF molecules and does not associate with the phosphorus lone pair or pendant charged carborane R-group (closest Cl distance to Li = 4.283 Å). This observation suggests that the phosphorus lone pair is available for binding other metals and that the weakly coordinating nature of the CB₁₁C₁₁ R-group remains intact.

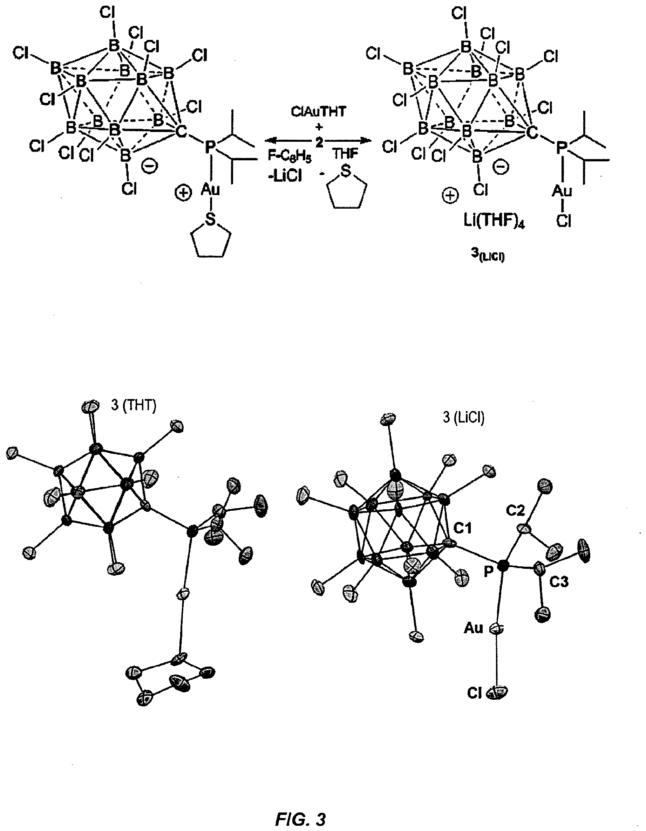

Anionic phosphine **2** (in Figure 1) was reacted with ClAu(THT) in fluorobenzene at RT, and within 5 minutes a large amount of white precipitate formed . Analysis of the precipitate by ³¹P NMR in CD₂Cl₂ shows a phosphorus resonance at +90 ppm, which is significantly shifted down field from the starting material **2** (+77 ppm), and in line with the formation of a Au-phosphine complex. The ¹H-NMR spectra clearly shows resonances corresponding to the phosphine ligand as well as coordinated THT, which suggests the formation of the zwitterionic Au complex **3_{(THT)}** (Figure 3) formed via ligand coordination and anion metathesis . The driving force for the anion metathesis is the insolubility of LiCl in F-benzene. A single crystal X-ray diffraction study confirms the structure of **3_{(THT)}**. If the reaction is performed in THF, a solvent where LiCl is soluble, THT is liberated and an unusual anionic ClAu-Li+ complex 3(LiCl) is isolated. Complex 3(LiCl) can also be prepared by the treatment of isolated 3(THT) with LiCl in THF. In contrast to 3(THT) it was possible to grow single crystals of 3 (LiCl) without significant disorder and thus examined the solid state structural aspects of this molecule. The Tolman cone angle for the AP-phosphine ligand is 204°, which indicates it is much more bulky than a standard sterically demanding phosphine ligand, such as P(t-Bu)3 (cone angle = 182°). The P-Au bond length is 2.2477(12) Å, which is close to a neutral ortho-dicarbaclosodecaborane substituted phosphine AuCl complex (2.232(3) Å). The anionic CB₁₁Cl₁₁ R-group displays one chloride interaction with the Au ion (closest Au....Cl-B distance = 3.118 Å), which is outside the sum of the covalent Au/Cl radii but in range of a van der Waals contact (sum of Au/Cl vdW radii = 3.41 Å). For comparison, the chloride ligand (trans from the phosphine) is at a distance of 2.2883(12) Å from the metal center. Hence, the carborane substituent retains a significant amount of weakly coordinating character, even though it is forced into a position close to the metal center.

### Example 16 - Hydroamination

**Table 1. Hydroamination of alkynes with primary amines and catalysts 3 (Figure 3).**

| | | | | | | | | | |
|---|---|---|---|---|---|---|---|---|---|
| 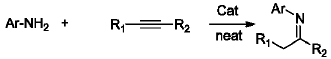 | | | | | | | | | |

| Entry | Cat | Ar | R₁ | R₂ | mol% | h | °C | % yield^{[a]} | T.O.N. |
|---|---|---|---|---|---|---|---|---|---|
| 1 | 3_{(THT)} | Ph | H | Ph | 0.1 | 1 | 25 | >95 | >950 |
| 2 | 3_{(LiCl)} | Ph | H | Ph | 0.1 | 1 | 25 | >95 | >950 |
| 3 | 3_{(THT)} | Ph | H | Ph | 0.01 | 16 | 50 | >95 | >9500 |
| 4 | 3_{(THT)} | Ph | H | Ph | 0.004 | 16 | 50 | 88 | 22,000 |
| 5 | 3_{(THT)} | Mes | H | Ph | 0.001 | 24 | 50 | 67 | 67,000 |
| 6 | 3_{(THT)} | Dipp | H | Ph | 0.001 | 24 | 50 | 85 | 85,000 |
| 7 | 3_{(THT)} | Ph | H | 4-FC₆H₄ | 0.001 | 24 | 50 | 54 | 54,000 |
| 8 | 3_{(THT)} | Mes | H | 4-FC₆H₄ | 0.001 | 24 | 50 | 75(60)^{[b]} | 75,000 |
| 9 | 3_{(THT)} | Dipp | H | 4-FC₆H₄ | 0.001 | 24 | 50 | 92 | 92,000 |
| 10 | 3_{(THT)} | Ph | H | 4-MeOC₆H₄ | 0.001 | 24 | 50 | 90 | 90,000 |
| 11 | 3_{(THT)} | Mes | H | 4-MeOC₆H₄ | 0.001 | 24 | 50 | 94(93)^{[b]} | 94,000 |
| 12 | 3_{(THT)} | Dipp | H | 4-MeOC₆H₄ | 0.001 | 24 | 50 | >95(88)^{[b]} | >95,000 |
| 13 | 3_{(THT)} | Ph | Ph | Ph | 0.1 | 24 | 80 | 89.5 | 895 |
| 14 | 3_{(THT)} | Mes | Ph | Ph | 0.1 | 24 | 80 | 67 | 670 |
| 15 | 3_{(THT)} | Dipp | Ph | Ph | 0.1 | 24 | 80 | 78.5 | 785 |
| 16 | 3_{(THT)} | Ph | H | *n*-C₄H₉ | 0.2 | 24 | 80 | 86.5 | 435 |
| 17 | 3_{(THT)} | Mes | H | *n*-C₄H₉ | 0.2 | 24 | 80 | 86 | 430 |
| 18 | 3_{(THT)} | Dipp | H | *n*-C₄H₉ | 0.2 | 24 | 80 | >95 | >475 |

Yields not in brackets, were measured by NMR via direct integration of the alkyne consumed versus the imine product formed, and reflect the average of two catalytic runs. No side reactions were observed. Number in brackets after the NMR yield indicate isolated yield.

This Example demonstrates the catalytic properties of complexes **3** in the hydroamination of primary amines with alkynes. This example observed the addition of aniline to phenyl acetylene. A 1:1 neat mixture of amine and alkyne was added to 0.1 mol% of complex **3**_{(THT)}, where upon an exothermic reaction occurred. Monitoring the reaction by ¹H NMR revealed the hydroamination was >95% complete in 1 hour (entry 1, Table 1). Identical results were obtained with 3(LiCl), (entry 2, Table 1), hence further catalytic tests were carried out using exclusively 3(THT). Reducing the catalyst loading to 0.01% and heating the mixture at 50°C for 16 hour does not reduce the yield of the imine (entry 3, table 1). Even at a catalyst loading of 0.004%, the imine is formed in excellent yield (88%), which corresponds to a catalyst turn over number (T.O.N.) of 22,000 (entry 4, table 1). The highest reported T.O.N. for the Au-catalyzed hydroamination of an alkyne with a primary amine is around 9000, using a multicomponent acid activated Au system. Control experiments with ClAu(THT), or HCB₁₁Cl₁₁-Cs+/ClAuTHT at low catalyst loading (0.1mol%) afforded the imine in only trace amounts (<5%). A metal free Bronsted acid catalyzed pathway can also be ruled out since (iPr)₂P(CB₁₁Cl₁₁)- H₃NPh⁺ does not catalyse the reaction. Since such high catalytic activity is extremely unusual for Au catalyzed reactions, this Example observed the hydroamination of phenyl acetylene with several different aryl amines at ultra-low loading (0.001%, 10 ppm). After 24 hours mesityl amine effectively reacts to afford the corresponding imine in 67% yield (T.O.N. = 67,000) (entry 5, table 1). Bulkier 2,6-diisopropylphenylamine (Dipp-amine) is even more efficient in the reaction, affording the analogous imine in 85% yield (T.O.N. = 85,000) (entry 6, table 1). For the latter reaction, the catalyst is also fast, displaying an initial Turn Over frequency of 29,000 in the first hour. Even higher T.O.N.s are obtained for the analogous reactions with 4-fluorophenylacetylene, reaching a maximum of 92,000 with Dipp-amine (entries 7-9, table 1). Results with 4-methoxyphenylacetylene were better than most, where the catalyst converted all three amines to the corresponding imines with T.O.N.s of 90,000 or greater (entries 10-12). The trend in increased reactivity with amine steric bulk is likely related to the decreased binding ability of bulkier amines, and the ensuing imines, which allows for more facile ligand exchange at the Au center. Catalyst 3(THT) is also effective for the hydroamination of aryl amines with diaryl substituted (entries 13-15, table 1), and terminal alkyl alkynes (entries 15-18, table 1). Although the activity is lower in these cases the performance of 3(THT) compares favorably with typical Au catalysts, which often require catalyst loadings of 1-10mol%. At low catalyst loading (0.2 mol%), and using internal dialkylalkynes (3-hexyne) or an alkyl amine (t-BuNH2) as substrates produced the expected imines only in trace amounts (<5%).

The two mechanisms generally proposed for Au catalyzed hydroamination either involve a direct addition of the amine to a coordinated alkyne or a coordination insertion mechanism. In either pathway, charged intermediates exist that undergo proton transfer steps that lead to the formation of the functionalized amine. It is clear that the pendant anionic CB₁₁Cl₁₁ group of AP-phosphine **2** is beneficial for the reaction sequence. If industrial scale applications are to be realized for homogenous Au catalysis, as well as systems utilizing other precious metals, it is necessary to have catalysts, such as complexes **3,** available that are highly active and efficient. Many transition metal catalyzed processes involve cationic intermediates that might benefit from the use of an AP-ligand.

## Claims

1. A ligand having the following structure:

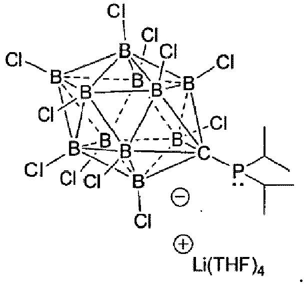

2. A complex having a structure selected from:

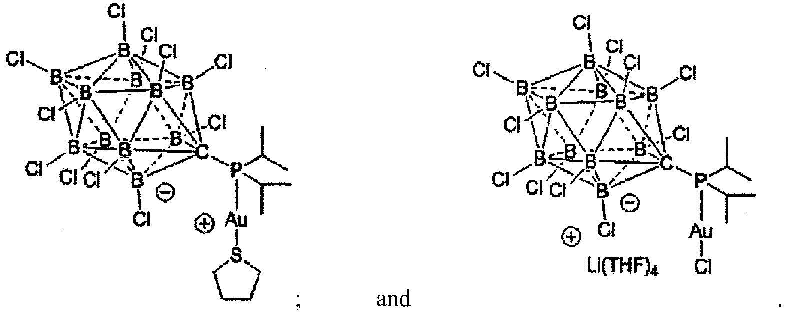

3. Use of the complex of claim 2 in a method of catalysing a chemical reaction.

4. The use of claim 3, wherein the chemical reaction is hydroamination.

5. A method for catalysing a chemical reaction comprising contacting the complex of claim 2 with suitable reagents for the chemical reaction to occur.

6. The method of claim 5, wherein the chemical reaction is a hydroamination reaction.

## Patentansprüche

1. Ligand, der die folgende Struktur aufweist:

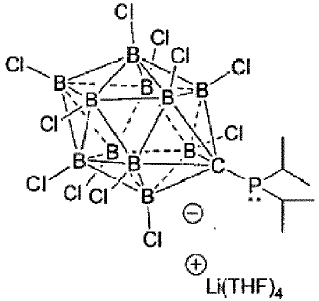

2. Komplex, der eine Struktur aufweist, die ausgewählt ist aus:

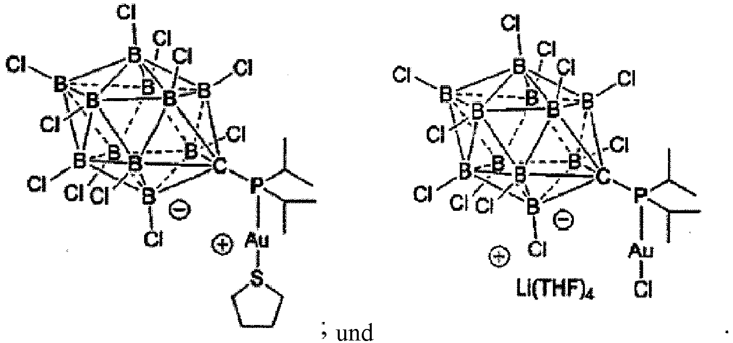

3. Verwendung des Komplexes von Anspruch 2 in einem Verfahren zum Katalysieren einer chemischen Reaktion.

4. Verwendung von Anspruch 3, wobei die chemische Reaktion Hydroaminierung ist.

5. Verfahren zu Katalysieren einer chemischen Reaktion, umfassend ein Inkontaktbringen des Komplexes von Anspruch 2 mit geeigneten Reagenzien, damit die chemische Reaktion auftritt.

6. Verfahren nach Anspruch 5, wobei die chemische Reaktion eine Hydroaminierungsreaktion ist.

## Revendications

1. Ligand ayant la structure suivante :

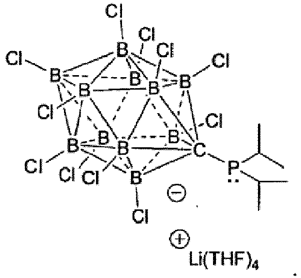

2. Complexe ayant une structure choisie parmi :

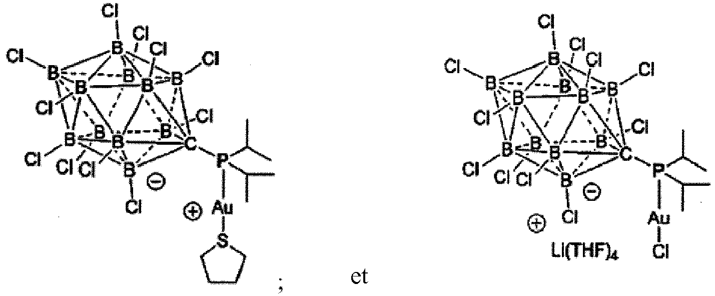

3. Utilisation du complexe de la revendication 2 dans un procédé de catalyse d'une réaction chimique.

4. Utilisation de la revendication 3, dans laquelle la réaction chimique est une hydroamination.

5. Procédé de catalyse d'une réaction chimique comprenant la mise en contact du complexe de la revendication 2 avec des réactifs appropriés pour permettre la survenue de la réaction chimique.

6. Procédé de la revendication 5, dans lequel la réaction chimique est une réaction d'hydroamination.
